(19) Europäisches Patentamt
European Patent Office
Office européen des brevets

(11) EP 1 029 052 B1

(12) **EUROPEAN PATENT SPECIFICATION**

(45) Date of publication and mention of the grant of the patent:
**04.08.2010 Bulletin 2010/31**

(21) Application number: **98946675.0**

(22) Date of filing: **09.10.1998**

(51) Int Cl.:
*C12N 15/31* (2006.01) *C07K 14/22* (2006.01)
*C07K 16/12* (2006.01) *G01N 33/53* (2006.01)
*A61K 39/095* (2006.01)

(86) International application number:
**PCT/IB1998/001665**

(87) International publication number:
**WO 1999/024578 (20.05.1999 Gazette 1999/20)**

(54) **NEISSERIAL ANTIGENS**

NEISSERIALE ANTIGENE

ANTIGENES DE NEISSERIA

(84) Designated Contracting States:
**AT BE CH CY DE DK ES FI FR GB GR IE IT LI LU MC NL PT SE**

(30) Priority:
**06.11.1997 GB 9723516**
**14.11.1997 GB 9724190**
**18.11.1997 GB 9724386**
**27.11.1997 GB 9725158**
**10.12.1997 GB 9726147**
**14.01.1998 GB 9800759**
**01.09.1998 GB 9819016**

(43) Date of publication of application:
**23.08.2000 Bulletin 2000/34**

(60) Divisional application:
**07075379.3 / 1 900 818**

(73) Proprietor: **Novartis Vaccines and Diagnostics S.r.l.**
**53100 Siena (SI) (IT)**

(72) Inventors:
- **MASIGNANI, Vega**
  **I-53100 Siena (IT)**
- **RAPPUOLI, Rino**
  **I-53019 Castelnuovo Berardenga (IT)**
- **PIZZA, Mariagrazia**
  **I-53100 Siena (IT)**
- **SCARLATO, Vincenzo**
  **I-53134 Colle Val d'Elsa (IT)**
- **GRANDI, Guido**
  **I-20090 Segrate (IT)**

(74) Representative: **Hallybone, Huw George**
**Carpmaels & Ransford**
**43-45 Bloomsbury Square**
**London WC1A 2RA (GB)**

(56) References cited:
**WO-A-96/12020** **WO-A-96/31618**
**WO-A-99/55873**

- **ROKBI, B. ET AL.: "Evaluation of Recombinant Transferrin-Binding Protein B Variants from Neisseria meningitidis for Their Ability To Induce Cross-Reactive and Bactericidal Antibodies against a Genetically Diverse Collection of Serogroup B Strains" INFECTION AND IMMUNITY, vol. 65, no. 1, January 1997 (1997-01), pages 55-63, XP002086937**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 029 052 B1

## Description

This invention relates to antigens from *Neisseria* bacteria.

## BACKGROUND ART

*Neisseria meningitidis* and *Neisseria gonorrhoeae* are non-motile, gram negative diplococci that are pathogenic in humans. *N.meningitidis* colonises the pharynx and causes meningitis (and, occasionally, septicaemia in the absence of meningitis); *N.gonorrhoeae* colonises the genital tract and causes gonorrhea. Although colonising different areas of the body and causing completely different diseases, the two pathogens are closely related, although one feature that clearly differentiates meningococcus from gonococcus is the presence of a polysaccharide capsule that is present in all pathogenic meningococci.

*N.gonorrhoeae* caused approximately 800,000 cases per year during the period 1983-1990 in the United States alone (chapter by Meitzner & Cohen, "Vaccines Against Gonococcal Infection", In: New Generation Vaccines, 2nd edition, ed. Levine, Woodrow, Kaper, & Cobon, Marcel Dekker, New York, 1997, pp.817-842). The disease causes significant morbidity but limited mortality. Vaccination against *N.gonorrhoeae* would be highly desirable, but repeated attempts have failed. The main candidate antigens for this vaccine are surface-exposed proteins such as pili, porins, opacity-associated proteins (Opas) and other surface-exposed proteins such as the Lip, Laz, IgAl protease and transferrin-binding proteins. The lipooligosaccharide (LOS) has also been suggested as vaccine (Meitzner & Cohen, *supra*).

*N.meningitidis* causes both endemic and epidemic disease. In the United States the attack rate is 0.6-1 per 100,000 persons per year, and it can be much greater during outbreaks (see Lieberman et al. (1996) Safety and Immunogenicity of a Serogroups A/C Neisseria meningitidis Oligosaccharide-Protein Conjugate Vaccine in Young Children. JAMA 275(19):1499-1503; Schuchat et al (1997) Bacterial Meningitis in the United States in 1995. NEngl J Med 337 (14):970-976). In developing countries, endemic disease rates are much higher and during epidemics incidence rates can reach 500 cases per 100,000 persons per year. Mortality is extremely high, at 10-20% in the United States, and much higher in developing countries. Following the introduction of the conjugate vaccine against *Haemophilus influenzae, N. meningitidis* is the major cause of bacterial meningitis at all ages in the United States (Schuchat *et al* (1997) *supra*).

Based on the organism's capsular polysaccharide, 12 serogroups of *N.meningitidis* have been identified. Group A is the pathogen most often implicated in epidemic disease in sub-Saharan Africa. Serogroups B and C are responsible for the vast majority of cases in the United States and in most developed countries. Serogroups W135 and Y are responsible for the rest of the cases in the United States and developed countries. The meningococcal vaccine currently in use is a tetravalent polysaccharide vaccine composed of serogroups A, C, Y and W135. Although efficacious in adolescents and adults, it induces a poor immune response and short duration of protection, and cannot be used in infants [eg. Morbidity and Mortality weekly report, Vol.46, No. RR-5 (1997)]. This is because polysaccharides are T-cell independent antigens that induce a weak immune response that cannot be boosted by repeated immunization. Following the success of the vaccination against *H.influenzae,* conjugate vaccines against serogroups A and C have been developed and are at the final stage of clinical testing (Zollinger WD "New and Improved Vaccines Against Meningococcal Disease" in: New Generation Vaccines, supra, pp. 469-488; Lieberman *et al* (1996) *supra;* Costantino et al (1992) Development and phase I clinical testing of a conjugate vaccine against meningococcus A and C. Vaccine 10:691-698).

Meningococcus B remains a problem, however. This serotype currently is responsible for approximately 50% of total meningitis in the United States, Europe, and South America. The polysaccharide approach cannot be used because the menB capsular polysaccharide is a polymer of $\alpha$(2-8)-linked N-acetyl neuraminic acid that is also present in mammalian tissue. This results in tolerance to the antigen; indeed, if an immune response were elicited, it would be anti-self, and therefore undesirable. In order to avoid induction of autoimmunity and to induce a protective immune response, the capsular polysaccharide has, for instance, been chemically modified substituting the N-acetyl groups with N-propionyl groups, leaving the specific antigenicity unaltered (Romero & Outschoorn (1994) Current status of Meningococcal group B vaccine candidates: capsular or non-capsular? Clin Microbiol Rev 7(4):559-575).

Alternative approaches to menB vaccines have used complex mixtures of outer membrane proteins (OMPs), containing either the OMPs alone, or OMPs enriched in porins, or deleted of the class 4 OMPs that are believed to induce antibodies that block bactericidal activity. This approach produces vaccines that are not well characterized. They are able to protect against the homologous strain, but are not effective at large where there are many antigenic variants of the outer membrane proteins. To overcome the antigenic variability, multivalent vaccines containing up to nine different porins have been constructed *(eg.* Poolman JT (1992) Development of a meningococcal vaccine. Infect. Agents Dis. 4: 13-28). Additional proteins to be used in outer membrane vaccines have been the opa and opc proteins, but none of these approaches have been able to overcome the antigenic variability (*eg.* Ala'Aldeen & Borriello (1996) The meningococcal transferrin-binding proteins 1 and 2 are both surface exposed and generate bactericidal antibodies capable of killing homologous and heterologous strains. Vaccine 14(1):49-53).

A certain amount of sequence data is available for meningococcal and gonoccocal genes and proteins (*eg.*

EP-A-0467714, WO96/29412), but this is by no means complete. The provision of further sequences could provide an opportunity to identify secreted or surface-exposed proteins that are presumed targets for the immune system and which are not antigenically variable. For instance, some of the identified proteins could be components of efficacious vaccines against meningococcus B, some could be components of vaccines against all meningococcal serotypes, and others could be components of vaccines against all pathogenic *Neisseriae.*

WO 9711181 describes the sequences of Neisserial adhesion proteins.

## THE INVENTION

The invention provides proteins comprising the Neisserial amino acid sequences disclosed in the examples. These sequences relate to *N.meningitidis* or *N.gonorrhoeae.*

It also provides proteins comprising sequences having 80% or greater sequence identity to the Neisserial amino acid sequences disclosed in the examples. Identity between the proteins is preferably determined by the Smith-Waterman homology search algorithm as implemented in the MPSRCH program (Oxford Molecular), using an affine gap search with parameters *gap open penalty = 12* and *gap extension penalty=1.*

The invention further provides proteins comprising fragments of the Neisserial amino acid sequences disclosed in the examples. The fragments comprise at least 20 consecutive amino acids from the sequences. The fragments comprise an epitope from the sequence.

The proteins of the invention can, of course, be prepared by various means (*eg*. recombinant expression, purification from cell culture, chemical synthesis *etc.*) and in various forms (*eg*. native, fusions *etc.*). They are preferably prepared in substantially pure or isolated form (*ie.* substantially free from other Neisserial or host cell proteins)

According to a further aspect, the invention provides nucleic acid comprising the Neisserial nucleotide sequences disclosed in the examples.

Furthermore, the invention provides nucleic acid which can hybridise to the Neisserial nucleic acid disclosed in the examples, preferably under "high stringency" conditions (*eg*. 65°C in a 0.1xSSC, 0.5% SDS solution).

It should also be appreciated that the invention provides nucleic acid comprising sequences complementary to those described above (*eg*. for antisense or probing purposes).

Nucleic acid according to the invention can, of course, be prepared in many ways (*eg*. by chemical synthesis, from genomic or cDNA libraries, from the organism itself *etc.*) and can take various forms (*eg*. single stranded, double stranded, vectors, probes *etc.*).

In addition, the term "nucleic acid" includes DNA and RNA, and also their analogues, such as those containing modified backbones, and also peptide nucleic acids (PNA) *etc.*

According to a further aspect, the invention provides compositions comprising protein, and/or nucleic acid according to the invention. These compositions may be suitable as vaccines, for instance, or as diagnostic reagents, or as immunogenic compositions.

The invention also provides nucleic acid, or protein according to the invention for use as medicaments (*eg*. as vaccines) or as diagnostic reagents. It also provides the use of nucleic acid, or protein, according to the invention in the manufacture of: a medicament for treating or preventing infection due to Neisserial bacteria. Said Neisserial bacteria may be any species or strain (such as *N.gonorrhoeae,* or any strain of *N.meningitidis,* such as strain A, strain B or strain C).

A summary of standard techniques and procedures which may be employed in order to perform the invention (*eg*. to utilise the disclosed sequences for vaccination or diagnostic purposes) follows. This summary is not a limitation on the invention but, rather, gives examples that may be used, but are not required.

### *General*

The practice of the present invention will employ, unless otherwise indicated, conventional techniques of molecular biology, microbiology, recombinant DNA, and immunology, which are within the skill of the art. Such techniques are explained fully in the literature *eg*. Sambrook Molecular Cloning; A Laboratory Manual, Second Edition (1989); DNA Cloning, Volumes I and ii (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C. C. Blackwell eds 1986).

Standard abbreviations for nucleotides and amino acids are used in this specification.

*Definitions*

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "comprising" means "including" as well as "consisting" *eg.* a composition "comprising" X may consist exclusively of X or may include something additional to X, such as X+Y.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Neisserial sequence is heterologous to a mouse host cell. A further examples would be two epitopes from the same or different proteins which have been assembled in a single protein in an arrangement not found in nature.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% *(eg.* 60%, 70%, 80%, 90%, 95%, 99% or more, calculated using the Smith-Waterman algorithm, as described above). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (*eg.* see US patent 5,753,235).

*Expression systems*

The Neisserial nucleotide sequences can be expressed in a variety of different expression systems; for example those used with mammalian cells, baculoviruses, plants, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (*eg.* structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.*].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237*;* Alberts et al. (1989) Molecular Biology of the Cell, 2nd ed.]. Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982b) Proc. Natl. Acad. Sci. 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*eg.* plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2, [Kaufman et al. (1989) Mol. Cell. Biol. 9:946] and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (*eg.* Hep G2), and a number of other cell lines.

ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit). These techniques are generally known to those skilled in the art and fully described in Summers and Smith, *Texas Agricultural Experiment Station Bulletin No. 1555* (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (*eg.* plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a prokaryotic ampicillin-resistance (*amp*) gene and origin of replication for selection and propagation in *E. coli*.

Baculovirus transfer vectors usually contain a baculovirus promoter. A baculovirus promoter is any DNA sequence capable of binding a baculovirus RNA polymerase and initiating the downstream (5' to 3') transcription of a coding sequence (*eg.* structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) *Gene, 73*:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human α-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra;* Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 μm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, *supra;* Miller et al. (1989).

Recombinant baculovirus expression vectors have been developed for infection into several insect cells. For example, recombinant baculoviruses have been developed for, *inter alia: Aedes aegypti , Autographa californica, Bombyx mori, Drosophila melanogaster, Spodoptera frugiperda,* and *Trichoplusia ni* (WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See, eg.* Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, eg. HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, eg. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iii. Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J. Biol. Chem. 260:3731-3738 (1985); Rothstein et al., Gene 55:353-356 (1987); Whittier et al., Nucleic Acids Research 15:2515-2535 (1987); Wirsel et al., Molecular Microbiology 3:3-14 (1989); Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolm B. Wilkins, ed., 1984 Pitman Publishing Limited, London, pp. 21-52. References that describe other metabolically-regulated genes: Sheen, Plant Cell, 2:1027-1038(1990); Maas et al., EMBO J. 9:3447-3452 (1990); Benkel and Hickey, Proc. Natl. Acad. Sci. 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation. (Fromm et al., Proc. Natl Acad. Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

iv. Bacterial Systems

Bacterial expression techniques are known in the art. A bacterial promoter is any DNA sequence capable of binding bacterial RNA polymerase and initiating the downstream (3') transcription of a coding sequence (*eg.* structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose *(lac) [*Chang et al. (1977) Nature 198:1056], and maltose. Additional examples include promoter sequences derived from biosynthetic enzymes such as tryptophan (*trp*) [Goeddel et al. (1980) Nuc. Acids Res. 8:4057; Yelverton et al. (1981) Nucl. Acids Res. 9:731; US patent 4,738,921; EP-A-0036776 and EP-A-0121775]. The g-laotamase (*bla*) promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad. Sci. 80:21].

Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an *E. coli* operator region (EPO-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon [Shine *et al.* (1975) *Nature 254:34].* The SD sequence is thought to promote binding of mRNA to the ribosome by the pairing of bases between the SD sequence and the 3' and of *E. coli* 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual*].*

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lacZ* [Jia et al. (1987) Gene 60:197], *trpE* [Allen et al. (1987) J. Biotechnol. 5:93; Makoff et al. (1989) J. Gen. Microbiol. 135:11], and Chey [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*eg.* ubiquitin specific processing-protease) to cleave the ubiquitin from the foreign protein. Through this method, native foreign protein can be isolated [Miller et al. (1989) Bio/ Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo or in vitro* encoded between the signal peptide fragment and the foreign gene.

DNA encoding suitable signal sequences can be derived from genes for secreted bacterial proteins, such as the *E. coli* outer membrane protein gene *(ompA)* [Masui et al. (1983), in: Experimental Manipulation of Gene Expression; Ghrayeb et al. (1984) EMBO J. 3:2437] and the *E. coli* alkaline phosphatase signal sequence *(phoA)* [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components; comprising a promoter, signal sequence (if desired), coding sequence of interest, and transcription termination sequence, are put together into expression constructs. Expression constructs are often maintained in a replicon, such as an extrachromosomal element (*eg*. plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: Bacillus subtilis [Palva et al. (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40: 183; Studier et al. (1986) J. Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. See *eg.* [Masson et al. (1989) FEMS Microbiol. Lett. 60:273; Palva et al. (1982) Proc. Natl. Acad. Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69: 2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbio/. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, Streptococcus].

v. Yeast Expression

Yeast expression systems are also known to one of ordinary skill in the art. A yeast promoter is any DNA sequence capable of binding yeast RNA polymerase and initiating the downstream (3') transcription of a coding sequence *(eg.* structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast *PHO5* gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880,734). Other examples of hybrid promoters include promoters which consist of the regulatory sequences of either the *ADH2, GAL4, GAL10,* OR *PHO5* genes, combined with the transcriptional activation region of a glycolytic enzyme gene such as GAP or PyK (EP-A-0 164 556). Furthermore, a yeast promoter can include naturally occurring promoters of non-yeast origin that have the ability to bind yeast RNA polymerase and initiate transcription. Examples of such promoters include, *inter alia,* [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See *eg.* EP-A-0 196 056. Another example is a ubiquitin fusion protein. Such a fusion protein is made with the ubiquitin region that preferably retains a site for a processing enzyme (*eg.* ubiquitin-specific processing protease) to cleave the ubiquitin from the foreign protein. Through this method, therefore, native foreign protein can be isolated (*eg.* WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EP-A-0 012 873; JPO. 62,096,086) and the A-factor gene (US patent 4,588,684). Alternatively, leaders of non-yeast origin, such as an interferon leader, exist that also provide for secretion in yeast (EP-A-0 060 057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (*eg.* see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (*eg.* plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/1 [Brake et al. (1984) Proc. Natl. Acad. Sci USA 81:4642-4646], and YRp17 [Stinchcomb et al. (1982) J. Mol. Biol. 158:157]. In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *eg.* Brake *et al., supra.*

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver *et al., supra.* One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRP1,* and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, *inter alia,* the following yeasts:Candida albicans [Kurtz, et al. (1986) Mol. Cell. Biol. 6:142], Candida maltosa [Kunze, et al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis *[*Das, et al. (1984) J. Bacteriol. 158:1165], Kluyveromyces lactis [De Louvencourt et al. (1983) J. Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5: 3376; US Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations. Transformation procedures usually vary with the yeast species to be transformed. See *eg.* [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165*;* De Louvencourt et al. (1983) J. Bacteriol. 154:1165; Van den Berg et al. (1990) BiolTechnology 8:135; Kluyveromyces]; [Cregg et al. (1985) Mol. Cell. Biol. 5: 3376; Kunze et al. (1985) J. Basic Microbiol. 25:141; US Patent Nos. 4,837,148 and 4,929,555; Pichia]; [Hinnen et al.

(1978) Proc. Natl. Acad. Sci. USA 75;1929; Ito et al. (1983) J. Bacteriol. 153:163 Saccharomyces]; [Beach and Nurse (1981) Nature 300:706*;* Schizosaccharomyces]; [Davidow et al. (1985) Curr. Genet. 10:39; Gaillardin et al. (1985) Curr. Genet. 10:49; Yarrowia].

*Pharmaceutical Compositions*

Pharmaceutical compositions can comprise either polypeptides, or nucleic acid of the invention. The pharmaceutical compositions will comprise a therapeutically effective amount of either polypeptides, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

*Delivery Methods*

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*eg.* see WO98/20734), needles, and gene guns or hyposprays. Dosage treatment may be a single dose schedule or a multiple dose schedule.

*Vaccines*

Vaccines according to the invention may either be prophylactic (*ie.* to prevent infection) or therapeutic (*ie.* to treat disease after infection).

Such vaccines comprise immunising antigen(s), immunogen(s), polypeptide(s), protein(s) or nucleic acid, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen or immunogen may be conjugated to a bacterial toxoid, such as a toxoid from diphtheria, tetanus, cholera, *H. pylori, etc.* pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59™ (WO 90/14837; Chapter 10 in *Vaccines design: the subunit and adjuvant approach,* eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (*eg.* IL-1, IL-2, IL-4, IL-5, IL-6, IL-7, IL-12, *etc.),* interferons *(eg.* gamma interferon), macrophage colony stimulating factor (M-CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N-acetyl-muramyl-L-threonyl-D-isoglutamie (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), *etc.*

The immunogenic compositions (*eg.* the immunising antigen/immunogen/polypeptide/protein/ nucleic acid, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated *(eg.* nonhuman primate, primate, *etc.*), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, *eg.* by injection, either subcutaneously, intramuscularly, or transdermally/transcutaneously (*eg.* WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

As an alternative to protein-based vaccines, DNA vaccination may be employed [*eg.* Robinson & Torres (1997) Seminars in Immunology 9:271-283; Donnelly et al. (1997) Annu Rev Immunol 15:617-648; see later herein].

## *Gene Delivery Vehicles*

Gene therapy vehicles for delivery of constructs including a coding sequence of a therapeutic of the invention, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences. The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153.

Retroviral vectors are well known in the art and we contemplate that any retroviral gene therapy vector is employable in the invention, including B, C and D type retroviruses, xenotropic retroviruses (for example, NZB-X1, NZB-X2 and NZB9-1 (see ONeill (1985) J. Viral. 53:160) polytropic retroviruses *eg.* MCF and MCF-MLV (see Kelly (1983) J. Virol. 45:291), spumaviruses and lentiviruses. See RNA Tumor Viruses, Second Edition, Cold Spring Harbor Laboratory, 1985.

Portions of the retroviral gene therapy vector may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

These recombinant retroviral vectors may be used to generate transduction competent retroviral vector particles by introducing them into appropriate packaging cell lines (see US patent 5,591,624). Retrovirus vectors can be constructed for site-specific integration into host cell DNA by incorporation of a chimeric integrase enzyme into the retroviral particle (see WO96/37626). It is preferable that the recombinant viral vector is a replication defective recombinant virus.

Packaging cell lines suitable for use with the above-described retrovirus vectors are well known in the art, are readily prepared (see WO95/30763 and WO92/05266), and can be used to create producer cell lines (also termed vector cell lines or "VCLs") for the production of recombinant vector particles. Preferably, the packaging cell lines are made from human parent cells *(eg.* HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

Exemplary known retroviral gene therapy vectors employable in this invention include those described in patent applications GB2200651, EP0415731, EP0345242, EP0334301, WO89/02468; WO89/05349, WO89/09271, WO90/02806, WO90/07936, WO94/03622, WO93/25698, WO93/25234, WO93/11230, WO93/10218, WO91/02805, WO91/02825, WO95/07994, US 5,219,740, US 4,405,712, US 4,861,719, US 4,980,289, US 4,777,127, US 5,591,624. See also Vile (1993) Cancer Res 53:3860-3864; Vile (1993) Cancer Res 53:962-967; Ram (1993) Cancer Res 53 (1993) 83-88; Takamiya (1992) J Neurosci Res 33:493-503; Baba (1993) J Neurosurg 79:729-735; Mann (1983) Cell 33:153; Cane (1984) Proc Natl Acad Sci 81:6349; and Miller (1990) Human Gene Therapy 1.

Human adenoviral gene therapy vectors are also known in the art and employable in this invention. See, for example, Berkner (1988) Biotechniques 6:616 and Rosenfeld (1991) Science 252:431, and WO93/07283, WO93/06223, and WO93/07282. Exemplary known adenoviral gene therapy vectors employable in this invention include those described in the above referenced documents and in WO94/12649, WO93/03769, WO93/19191, WO94/28938, WO95/11984, WO95/00655, WO95/27071, WO95/29993, WO95/34671, WO96/05320, WO94/08026, WO94/11506, WO93/06223, WO94/24299, WO95/14102, WO95/24297, WO95/02697, WO94/28152, WO94/24299, WO95/09241, WO95/25807, WO95/05835, WO94/18922 and WO95/09654. Alternatively, administration of DNA linked to killed adenovirus as described in Curiel (1992) Hum. Gene Ther. 3:147-154 may be employed. The gene delivery vehicles of the invention also include adenovirus associated virus (AAV) vectors. Leading and preferred examples of such vectors for use in this invention are the AAV-2 based vectors disclosed in Srivastava, WO93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 native nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (*ie.* there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, both of which are disclosed in Nahreini (1993) Gene 124:257-262. Another example of such an AAV vector is psub201 (see Samulski (1987) J. Virol. 61:3096). Another exemplary AAV vector is the Double-D ITR vector. Construction of the Double-D ITR vector is disclosed in US Patent 5,478,745. Still other vectors are those disclosed in Carter US Patent 4,797,368 and Muzyczka US Patent 5,139,941, Chartejee US Patent 5,474,935, and Kotin WO94/288157. Yet a further example of an AAV vector employable in this invention is SSV9AFABTKneo, which contains the AFP enhancer and albumin promoter and directs expression predominantly in the liver. Its structure and construction are disclosed in Su (1996) Human Gene Therapy 7:463-470. Additional AAV gene therapy vectors are described in US 5,354,678, US 5,173,414, US 5,139,941, and US 5,252,479.

The gene therapy vectors of the invention also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar Institute), pHSVlac described in Geller (1988) Science 241:1667-1669 and in WO90/09441 and WO92/07945, HSV Us3::pgC-lacZ described in Fink (1992) Human Gene Therapy 3:11-19 and HSV 7134, 2 RH 105 and GAL4 described in EP 0453242 (Breakefield), and those deposited with the ATCC as accession numbers ATCC VR-977 and ATCC VR-260.

Also contemplated are alpha virus gene therapy vectors that can be employed in this invention. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in US Serial No. 08/405,627, filed March 15, 1995, WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukarytic layered expression systems are also useful for expressing the nucleic acids of the invention. See WO95/07994 for a detailed description of eukaryotic layered expression systems. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) J. Biol. Standardization 1:115; rhinovirus, for example ATCC VR-1110 and those described in Arnold (1990) J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) Vaccine 8:17; in US 4,603,112 and US 4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J. Viol 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR-65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell deliver vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) Mol Cell Biol 14: 2411-2418 and in Woffendin (1994) Proc Natl Acad Sci 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin.

Naked DNA may also be employed. Exemplary naked DNA introduction methods are described in WO 90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in US 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al (1994) Proc. Natl. Acad. Sci. USA 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hard-held gene transfer particle gun, as described in US 5,149,655; use of ionizing radiation for activating transferred gene, as described in US 5,206,152 and WO92/1103

Exemplary liposome and polycationic gene delivery vehicles are those described in US 5,422,120 and 4,762,915; inWO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprises therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

<u>*Delivery Methods*</u>

Once formulated, the polynucleotide compositions of the invention can be administered (1) directly to the subject; (2) delivered *ex vivo,* to cells derived from the subject; or (3) *in vitro* for expression of recombinant proteins. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*eg*. see WO98/20734), needles, and gene guns or hyposprays. Dosage treatment may be a single dose schedule or a multiple dose schedule.

Methods for the *ex vivo* delivery and reimplantation of transformed cells into a subject are known in the art and described in *eg*. WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

<u>*Polynucleotide and polypeptide pharmaceutical compositions*</u>

In addition to the pharmaceutically acceptable carriers and salts described above, the following additional agents can be used with polynucleotide and/or polypeptide compositions.

<u>A.Polypeptides</u>

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

<u>B.Hormones, Vitamins, *etc.*</u>

Other groups that can be included are, for example: hormones, steroids, androgens, estrogens, thyroid hormone, or vitamins, folic acid.

C.Polyalkylenes, Polysaccharides, *etc.*

Also, polyalkylene glycol can be included with the desired polynucleotides/polypeptides. In a preferred embodiment, the polyalkylene glycol is polyethlylene glycol. In addition, mono-, di-, or polysaccarides can be included. In a preferred embodiment of this aspect, the polysaccharide is dextran or DEAE-dextran. Also, chitosan and poly(lactide-co-glycolide)

D.Lipids, and Liposomes

The desired polynucleotide/polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) Proc. Natl. Acad. Sci. USA 84:7413-7416); mRNA (Malone (1989) Proc. Natl. Acad. Sci. USA 86: 6077-6081); and purified transcription factors (Debs (1990) J. Biol. Chem. 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner *supra*). Other commercially available liposomes include transfectace (DDAB/DOPE) and DOTAP/DOPE (Boerhinger). Other cationic liposomes can be prepared from readily available materials using techniques well known in the art. See, *eg.* Szoka (1978) Proc. Natl. Acad. Sci. USA 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art t.

The liposomes can comprise multilammelar vesicles (MLVs), small unilamellar vesicles (SUVs), or large unilamellar vesicles (LUVs). The various liposome-nucleic acid complexes are prepared using methods known in the art. See *eg.* Straubinger (1983) Meth. Immunol. 101:512-527; Szoka (1978) Proc. Natl. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

E.Lipoproteins

In addition, lipoproteins can be included with the polynucleotide/polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C, and E, over time these lipoproteins lose A and acquire C and E apoproteins. VLDL comprises A, B, C, and E apoproteins, LDL comprises apoprotein B; and HDL comprises apoproteins A, C, and E.

The amino acid of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phopholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described *in Meth. Enzymol. (supra);* Pitas (1980) J. Biochem. 255:5454-5460 and Mahey (1979) J Clin. Invest 64: 743-750. Lipoproteins can also be produced by *in vitro* or recombinant methods by expression of the apoprotein genes in a desired host cell. See, for example, Atkinson (1986) Annu Rev Biophys Chem 15:403 and Radding (1958) Biochim Biophys Acta 30: 443. Lipoproteins can also be purchased from commercial suppliers, such as Biomedical Technologies, Inc., Stoughton, Massachusetts, USA. Further description of lipoproteins can be found in Zuckermann *et al.* PCT/L1S97/14465.

F.Polycationic Agents

Polycationic agents can be included, with or without lipoprotein, in a composition with the desired polynucleotide/polypeptide to be delivered.

Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.

The following are examples of useful polypeptides as polycationic agents: polylysine, polyarginine, polyornithine, and protamine. Other examples include histones, protamines, human serum albumin, DNA binding proteins, non-histone chromosomal proteins, coat proteins from DNA viruses, such as (X174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.

Organic polycationic agents include: spermine, spermidine, and purtrescine.

The dimensions and of the physical properties of a polycationic agent can be extrapolated from the list above, to construct other polypeptide polycationic agents or to produce synthetic polycationic agents.

Synthetic polycationic agents which are useful include, for example, DEAE-dextran, polybrene. Lipofectin™, and lipofectAMINE™ are monomers that form polycationic complexes when combined with polynucleotides/polypeptides.

*Immunodiagnostic Assays*

Neisserial antigens of the invention can be used in immunoassays to detect antibody levels (or, conversely, anti-Neisserial antibodies can be used to detect antigen levels). Immunoassays based on well defined, recombinant antigens can be developed to replace invasive diagnostics methods. Antibodies to Neisserial proteins within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, *etc.*) required for the conduct of the assay, as well as suitable set of assay instructions.

*Nucleic Acid Hybridisation*

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al.* [*supra*] Volume 2, chapter 9, pages 9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al.* at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1$\mu$g for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 $\mu$g of yeast DNA, blotting for two hours, and hybridizing for 4-8 hours with a probe of $10^8$ cpm/$\mu$g. For a single-copy mammalian gene a conservative approach would start with 10 $\mu$g of DNA, blot overnight, and hybridize overnight in the presence of 10% dextran sulfate using a probe of greater than $10^8$ cpm/$\mu$g, resulting in an exposure time of~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation:

$$Tm = 81 + 16.6(\log_{10}Ci) + 0.4[\%(G + C)] - 0.6(\%formamide) - 600/n - 1.5(\%mismatch).$$

where Ci is the salt concentration (monovalent ions) and n is the length of the hybrid in base pairs (slightly modified from Meinkoth & Wahl (1984) Anal. Biochem. 138: 267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (*ie*. stringency), it becomes less likely for hybridization to occur between strands that are nonhomologous, and as a result, background decreases. If the radiolabeled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

*Nucleic Acid Probe Assays*

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the Neisserial nucleotide sequences of the invention (including both sense and antisense strands). Though many different nucleotide sequences will encode the amino acid sequence, the native Neisserial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Neisserial sequence (or its complement) - some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Neisserial sequence may also be helpful as a label to detect the formed duplex. For example, a non-complementary nucleotide sequence may be attached to the 5' end of the probe, with the remainder of the probe sequence being complementary to a Neisserial sequence. Alternatively, non-complementary bases or longer sequences can be interspersed into the probe, provided that the probe sequence has sufficient complementarity with the a Neisserial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably at least 30 nucleotides, although it may be shorter than this. Short primers generally require cooler temperatures to form sufficiently stable hybrid complexes with the template.

Probes may be produced by synthetic procedures, such as the triester method of Matteucci et al. [J. Am. Chem. Soc. (1981) 103:3185], or according to Urdea et al. [Proc. Natl. Acad. Sci. USA (1983) 80: 7461], or using commercially available automated oligonucleotide synthesizers.

The chemical nature of the probe can be selected according to preference. For certain applications, DNA or RNA are appropriate. For other applications, modifications may be incorporated *eg*. backbone modifications, such as phosphorothioates or methylphosphonates, can be used to increase *in vivo* half-life, alter RNA affinity, increase nuclease resistance *etc.* [*eg.* see Agrawal & Iyer (1995) Curr Opin Biotechnol 6:12-19*;* Agrawal (1996) TIBTECH 14:376-387]; analogues such as peptide nucleic acids may also be used [*eg.* see Corey (1997) TIBTECH 15:224-229; Buchardt et al. (1993) TIBTECH 11:384-386].

Alternatively, the polymerase chain reaction (PCR) is another well-known means for detecting small amounts of target nucleic acids. The assay is described in: Mullis et al. [Meth. Enzymol. (1987) 155: 335-350]; US patents 4,683,195 and 4,683,202. Two "primer" nucleotides hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Neisserial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labelled probe will hybridize to the Neisserial sequence (or its complement).

Also, mRNA or cDNA can be detected by traditional blotting techniques described in Sambrook *et al* [*supra*]. mRNA, or cDNA generated from mRNA using a polymerase enzyme, can be purified and separated using gel electrophoresis. The nucleic acids on the gel are then blotted onto a solid support, such as nitrocellulose. The solid support is exposed to a labelled probe and then washed to remove any unhybridized probe. Next, the duplexes containing the labeled probe are detected. Typically, the probe is labelled with a radioactive moiety.

## EXAMPLES

The examples describe nucleic acid sequences which have been identified in *N.meningitidis,* along with their putative translation products, and also those of *N.gonorrhoeae.* Not all of the nucleic acid sequences are complete ie. they encode less than the full-length wild-type protein.

The examples are generally in the following format:

- a nucleotide sequence which has been identified in *N.meningitidis* (strain B)
- the putative translation product of this sequence
- a computer analysis of the translation product based on database comparisons
- corresponding gene and protein sequences identified in *N.meningitidis* (strain A) and in *N.gonorrhoeae*
- a description of the characteristics of the proteins which indicates that they might be suitably antigenic
- results of biochemical analysis (expression, purification, ELISA, FACS *etc.*)

The examples typically include details of sequence identity between species and strains. Proteins that are similar in sequence are generally similar in both structure and function, and the sequence identity often indicates a common evolutionary origin. Comparison with sequences of proteins of known function is widely used as a guide for the assignment of putative protein function to a new sequence and has proved particularly useful in whole-genome analyses.

Sequence comparisons were performed at NCBI (http://www.ncbi.nlm.nih.gov) using the algorithms BLAST, BLAST2, BLASTn, BLASTp, tBLASTn, BLASTx, & tBLASTx [*eg.* see also Altschul et al. (1997) Gapped BLAST and PSI-BLAST: a new generation of protein database search programs. Nucleic Acids Research 25:2289-3402]. Searches were performed against the following databases: non-redundant GenBank+EMBL+DDBJ+PDB sequences and non-redundant GenBank CDS translations+PDB+SwissProt+SPupdate+PIR sequences.

To compare Meningococcal and Gonococcal sequences, the tBLASTx algorithm was used, as implemented at http://www.genomc.ou.edu/gono_blast.html. The FASTA algorithm was also used to compare the ORFs (from GCG Wisconsin Package, version 9.0).

Dots within nucleotide sequences represent nucleotides which have been arbitrarily introduced in order to maintain a reading frame. In the same way, double-underlined nucleotides were removed. Lower case letters represent ambiguities which arose during alignment of independent sequencing reactions (some of the nucleotide sequences in the examples are derived from combining the results of two or more experiments).

Nucleotide sequences were scanned in all six reading frames to predict the presence of hydrophobic domains using an algorithm based on the statistical studies of Esposti et al. [Critical evaluation of the hydropathy of membrane proteins (1990) Eur J Biochem 190:207-219]. These domains represent potential transmembrane regions or hydrophobic leader sequences.

Open reading frames were predicted from fragmented nucleotide sequences using the program ORFFINDER (NCBI).

Underlined amino acid sequences indicate possible transmembrane domains or leader sequences in the ORFs, as predicted by the PSORT algorithm (http://www.psort.nibb.ac.jp). Functional domains were also predicted using the MOTIFS program (GCG Wisconsin & PROSITE).

Various tests can be used to assess the *in vivo* immunogencity of the proteins identified in the examples. For example, the proteins can be expressed recombinantly and used to screen patient sera by immunoblot. A positive reaction between the protein and patient serum indicates that the patient has previously mounted an immune response to the protein in question ie. the protein is an immunogen. This method can also be used to identify immunodominant proteins.

The recombinant protein can also be conveniently used to prepare antibodies *eg*. in a mouse. These can be used for direct confirmation that a protein is located on the cell-surface. Labelled antibody (*eg*. fluorescent labelling for FACS) can be incubated with intact bacteria and the presence of label on the bacterial surface confirms the location of the protein.

In particular, the following methods (A) to (S) were used to express, purify and biochemically characterise the proteins of the invention:

**A) Chromosomal DNA preparation**

*N.meningitidis* strain 2996 was grown to exponential phase in 100ml of GC medium, harvested by centrifugation, and resuspended in 5ml buffer (20% Sucrose, 50mM Tris-HCl, 50mM EDTA, pH8). After 10 minutes incubation on ice, the bacteria were lysed by adding 10ml lysis solution (50mM NaCl, 1% Na-Sarkosyl, 50μg/ml Proteinase K), and the suspension was incubated at 37°C for 2 hours. Two phenol extractions (equilibrated to pH 8) and one $ChCl_3$/isoamylalcohol (24:1) extraction were performed. DNA was precipitated by addition of 0.3M sodium acetate and 2 volumes ethanol, and was collected by centrifugation. The pellet was washed once with 70% ethanol and redissolved in 4ml buffer (10mM Tris-HCl, 1mM EDTA, pH 8). The DNA concentration was measured by reading the OD at 260 nm.

**B) Oligonucleotide design**

Synthetic oligonucleotide primers were designed on the basis of the coding sequence of each ORF, using (a) the meningococcus B sequence when available, or (b) the gonococcus/meningococcus A sequence, adapted to the codon preference usage of meningococcus as necessary. Any predicted signal peptides were omitted, by deducing the 5'-end amplification primer sequence immediately downstream from the predicted leader sequence.

For most ORFs, the 5' primers included two restriction enzyme recognition sites *(BamH*I-*Nde*I*, BamH*I-*Nhe*I*,* or *EcoR*I-*Nhe*I*,* depending on the gene's own restriction pattern); the 3' primers included a *Xho*I restriction site. This procedure was established in order to direct the cloning of each amplification product (corresponding to each ORF) into two different expression systems: pGEX-KG (using either *BamH*I-*Xho*I or *EcoR*I-*Xho*I*),* and pET21b+ (using either *Nde*I-*Xho*I or *Nhe*I-*Xho*I*).*

| | | |
|---|---|---|
| 5'-end primer tail: | CGC<u>GGATCCCATATG</u> | (*BamH*I-*Nde*I) |
| | CGC<u>GGATCCGCTAGC</u> | (*BamH*I-*Nhe*I*)* |
| | CCG<u>GAATTC</u>TA<u>GCTAGC</u> | (*EcoR*I-*Nhe*I |
| 3'-end primer tail: | CCCG<u>CTCGAG</u> | (*Xho*I) |

As well as containing the restriction enzyme recognition sequences, the primers included nucleotides which hybridizeed to the sequence to be amplified. The number of hybridizing nucleotides depended on the melting temperature of the whole primer, and was determined for each primer using the formulae:

$$T_m = 4\,(G+C) + 2\,(A+T) \qquad \text{(tail excluded)}$$

$$T_m = 64.9 + 0.41\,(\%\,GC) - 600/N \qquad \text{(whole primer)}$$

The average melting temperature of the selected oligos were 65-70°C for the whole oligo and 50-55°C for the hybridising region alone.

Oligos were synthesized by a Perkin Elmer 394 DNA/RNA Synthesizer, eluted from the columns in 2ml $NH_4OH$, and deprotected by 5 hours incubation at 56°C. The oligos were precipitated by addition of 0.3M Na-Acetate and 2 volumes ethanol. The samples were then centrifuged and the pellets resuspended in either 100μl or 1ml of water. $OD_{260}$ was determined using a Perkin Elmer Lambda Bio spectophotometer and the concentration was determined and adjusted to 2-10μmol/μl.

**C) Amplification**

The standard PCR protocol was as follows: 50-200ng of genomic DNA were used as a template in the presence of 20-40μM of each oligo, 400-800μM dNTPs solution, 1x PCR buffer (including 1.5mM $MgCl_2$), 2.5 units *Taq*I DNA polymerase (using Perkin-Elmer AmpliTaQ, GIBCO Platinum, Pwo DNA polymerase, or Tahara Shuzo Taq polymerase).

In some cases, PCR was optimsed by the addition of 10μl DMSO or 50μl 2M betaine.

After a hot start (adding the polymerase during a preliminary 3 minute incubation of the whole mix at 95°C), each sample underwent a double-step amplification: the first 5 cycles were performed using as the hybridization temperature the one of the oligos excluding the restriction enzymes tail, followed by 30 cycles performed according to the hybridization temperature of the whole length oligos. The cycles were followed by a final 10 minute extension step at 72°C.

The standard cycles were as follows:

| | Denaturation | Hybridisation | Elongation |
|---|---|---|---|
| First 5 cycles | 30 seconds 95°C | 30 seconds 50-55°C | 30-60 seconds 72°C |
| Last 30 cycles | 30 seconds 95°C | 30 seconds 65-70°C | 30-60 seconds 72°C |

The elongation time varied according to the length of the ORF to be amplified.

The amplifications were performed using either a 9600 or a 2400 Perkin Elmer GeneAmp PCR System. To check the results, 1/10 of the amplification volume was loaded onto a 1-1.5% agarose gel and the size of each amplified fragment compared with a DNA molecular weight marker.

The amplified DNA was either loaded directly on a 1% agarose gel or first precipitated with ethanol and resuspended in a suitable volume to be loaded on a 1% agarose gel. The DNA fragment corresponding to the right size band was then eluted and purified from gel, using the Qiagen Gel Extraction Kit, following the instructions of the manufacturer. The final volume of the DNA fragment was 30μl or 50μl of either water or 10mM Tris, pH 8.5.

**D) Digestion of PCR fragments**

The purified DNA corresponding to the amplified fragment was split into 2 aliquots and double-digested with:

- *Nde*I/*Xho*I or *Nhe*I/*Xho*I for cloning into pET-21b+ and further expression of the protein as a C-terminus His-tag fusion
- *BamH*I/*XhoI or EcoRI*/*XhoI* for cloning into pGEX-KG and further expression of the protein as N-terminus GST fusion.
- *EcoRI*/*PstI, EcoRI*/*SalI, SalI*/*Pst*I for cloning into pGex-His and further expression of the protein as N-terminus His-tag fusion

Each purified DNA fragment was incubated (37°C for 3 hours to overnight) with 20 units of each restriction enzyme (New England Biolabs) in a either 30 or 40μl final volume in the presence of the appropriate buffer. The digestion product was then purified using the QIAquick PCR purification kit, following the manufacturer's instructions, and eluted in a final volume of 30 or 50μl of either water or 10mM Tris-HCl, pH 8.5. The final DNA concentration was determined by 1% agarose gel electrophoresis in the presence of titrated molecular weight marker.

**E) Digestion of the cloning vectors (pET22B, pGEX-KG, pTRC-His A, and pGex-His)**

10μg plasmid was double-digested with 50 units of each restriction enzyme in 200μl reaction volume in the presence of appropriate buffer by overnight incubation at 37°C. After loading the whole digestion on a 1% agarose gel, the band corresponding to the digested vector was purified from the gel using the Qiagen QIAquick Gel Extraction Kit and the DNA was eluted in 50μl of 10mM Tris-HCl, pH 8.5. The DNA concentration was evaluated by measuring $OD_{260}$ of the sample, and adjusted to 50μg/μl. 1μl of plasmid was used for each cloning procedure.

The vector pGEX-His is a modified pGEX-2T vector carrying a region encoding six histidine residues upstream to the thrombin cleavage site and containing the multiple cloning site of the vector pTRC99 (Pharmacia).

**F) Cloning**

The fragments corresponding to each ORF, previously digested and purified, were ligated in both pET22b and pGEX-KG. In a final volume of 20μl, a molar ratio of 3:1 fragment/vector was ligated using 0.5μl of NEB T4 DNA ligase (400 units/μl), in the presence of the buffer supplied by the manufacturer. The reaction was incubated at room temperature for 3 hours. In some experiments, ligation was performed using the Boheringer "Rapid Ligation Kit", following the manufacturer's instructions.

In order to introduce the recombinant plasmid in a suitable strain, 100μl *E. coli* DH5 competent cells were incubated with the ligase reaction solution for 40 minutes on ice, then at 37°C for 3 minutes, then, after adding 800μl LB broth, again at 37°C for 20 minutes. The cells were then centrifuged at maximum speed in an Eppendorf microfuge and resuspended in approximately 200μl of the supernatant. The suspension was then plated on LB ampicillin (100mg/ml).

The screening of the recombinant clones was performed by growing 5 randomly-chosen colonies overnight at 37°C in either 2ml (pGEX or pTC clones) or 5ml (pET clones) LB broth + 100μg/ml ampicillin. The cells were then pelletted and the DNA extracted using the Qiagen QIAprep Spin Miniprep Kit, following the manufacturer's instructions, to a final volume of 30μl, 5μl of each individual miniprep (approximately 1g) were digested with either *Nde*I/*Xho*I or *BamH*I/*Xho*I and the whole digestion loaded onto a 1-1.5% agarose gel (depending on the expected insert size), in parallel with the molecular weight marker (1Kb DNA Ladder, GIBCO). The screening of the positive clones was made on the base of the correct insert size.

**G) ELISA assay (sera analysis)**

The acapsulated MenB M7 strain was plated on chocolate agar plates and incubated overnight at 37°C. Bacterial colonies were collected from the agar plates using a sterile dracon swab and inoculated into 7ml of Mueller-Hinton Broth (Difco) containing 0.25% Glucose. Bacterial growth was monitored every 30 minutes by following $OD_{620}$. The bacteria were let to grow until the OD reached the value of 0.3-0.4. The culture was centrifuged for 10 minutes at 10000rpm. The supernatant was discarded and bacteria were washed once with PBS, resuspended in PBS containing 0.025% formaldehyde, and incubated for 2 hours at room temperature and then overnight at 4°C with stirring. 100μl bacterial cells were added to each well of a 96 well Greiner plate and incubated overnight at 4°C. The wells were then washed three times with PBT washing buffer (0.1% Tween-20 in PBS). 200μl of saturation buffer (2.7% Polyvinylpyrrolidone 10 in water) was added to each well and the plates incubated for 2 hours at 37°C. Wells were washed three times with PBT. 200μl of diluted sera (Dilution buffer: 1% BSA, 0.1% Tween-20, 0.1% $NaN_3$ in PBS) were added to each well and the plates incubated for 90 minutes at 37°C. Wells were washed three times with PBT. 100μl of HRP-conjugated rabbit anti-mouse (Dako) serum diluted 1:2000 in dilution buffer were added to each well and the plates were incubated for 90 minutes at 37°C. Wells were washed three times with PBT buffer. 100μl of substrate buffer for HRP (25ml of citrate buffer pH5, 10mg of O-phenildiamine and 10μl of $H_2O$) were added to each well and the plates were left at room temperature for 20 minutes. 100μl $H_2SO_4$ was added to each well and $OD_{490}$ was followed. The ELISA was considered positive when $OD_{490}$ was 2.5 times the respective pre-immune sera.

**H) FACScan bacteria Binding Assay procedure.**

The acapsulated MenB M7 strain was plated on chocolate agar plates and incubated overnight at 37°C. Bacterial colonies were collected from the agar plates using a sterile dracon swab and inoculated into 4 tubes containing 8ml each Mueller-Hinton Broth (Difco) containing 0.25% glucose. Bacterial growth was monitored every 30 minutes by following $OD_{620}$. The bacteria were let to grow until the OD reached the value of 0.35-0.5. The culture was centrifuged for 10 minutes at 4000rpm. The supernatant was discarded and the pellet was resuspended in blocking buffer (1% BSA, 0.4% $NaN_3$) and centrifuged for 5 minutes at 4000rpm. Cells were resuspended in blocking buffer to reach $OD_{620}$ of 0.07. 100μl bacterial cells were added to each well of a Costar 96 well plate. 100ul of diluted (1:200) sera (in blocking buffer) were added to each well and plates incubated for 2 hours at 4°C. Cells were centrifuged for 5 minutes at 4000rpm, the supernatant aspirated and cells washed by addition of 200μl/well of blocking buffer in each well. 100μl of R-Phicoerytrin conjugated $F(ab)_2$ goat anti-mouse, diluted 1:100, was added to each well and plates incubated for 1 hour at 4°C. Cells were spun down by centrifugation at 4000rpm for 5 minutes and washed by addition of 200μl/well of blocking buffer. The supernatant was aspirated and cells resuspended in 200μl/well of PBS, 0.25% formaldehyde. Samples were transferred to FACScan tubes and read. The condition for FACScan setting were: FL1 on, FL2 and FL3 off; FSC-H threshold:92; FSC PMT Voltage: E 02; SSC PMT: 474; Amp. Gains 7.1; FL-2 PMT: 539; compensation values: 0.

**I) OMV preparations**

Bacteria were grown overnight on 5 GC plates, harvested with a loop and resuspended in 10 ml 20mM Tris-HCl. Heat inactivation was performed at 56°C for 30 minutes and the bacteria disrupted by sonication for 10 minutes on ice (50% duty cycle, 50% output). Unbroken cells were removed by centrifugation at 5000g for 10 minutes and the total cell envelope fraction recovered by centrifugation at 50000g at 4°C for 75 minutes. To extract cytoplasmic membrane proteins from the crude outer membranes, the whole fraction was resuspended in 2% sarkosyl (Sigma) and incubated at room temperature for 20 minutes. The suspension was centrifuged at 10000g for 10 minutes to remove aggregates, and the supernatant further ultracentrifuged at 50000g for 75 minutes to pellet the outer membranes. The outer membranes were resuspended in 10mM Tris-HCl, pH8 and the protein concentration measured by the Bio-Rad Protein assay, using BSA as a standard.

**J) Whole Extracts preparation**

Bacteria were grown overnight on a GC plate, harvested with a loop and resuspended in 1ml of 20mM Tris-HCl. Heat inactivation was performed at 56°C for 30 minutes.

**K) Western blotting**

Purified proteins (500ng/lane), outer membrane vesicles (5μg) and total cell extracts (25μg) derived from MenB strain 2996 were loaded on 15% SDS-PAGE and transferred to a nitrocellulose membrane. The transfer was performed for 2 hours at 150mA at 4°C, in transferring buffer 0.3% Tris base, 1.44 % glycine, 20% methanol). The membrane was saturated by overnight incubation at 4°C in saturation buffer (10% skimmed milk, 0.1% Triton X100 in PBS). The membrane was washed twice with washing buffer (3% skimmed milk, 0.1% Triton X100 in PBS) and incubated for 2 hours at 37°C with mice sera diluted 1:200 in washing buffer. The membrane was washed twice and incubated for 90 minutes with a 1:2000 dilution of horseradish peroxidase labelled anti-mouse Ig. The membrane was washed twice with 0.1% Triton X100 in PBS and developed with the Opti-4CN Substrate Kit (Bio-Rad). The reaction was stopped by adding water.

**L) Bactericidal assay**

MC58 strain was grown overnight at 37°C on chocolate agar plates. 5-7 colonies were collected and used to inoculate 7ml Mueller-Hinton broth. The suspension was incubated at 37°C on a nutator and let to grow until $OD_{620}$ was 0.5-0.8. The culture was aliquoted into sterile 1.5ml Eppendorf tubes and centrifuged for 20 minutes at maximum speed in a microfuge. The pellet was washed once in Gey's buffer (Gibco) and resuspended in the same buffer to an $OD_{620}$ of 0.5, diluted 1:20000 in Gey's buffer and stored at 25°C.

50μl of Gey's buffer/1% BSA was added to each well of a 96-well tissue culture plate. 25μl of diluted mice sera (1:100 in Gey's buffer/0.2% BSA) were added to each well and the plate incubated at 4°C. 25μl of the previously described bacterial suspension were added to each well. 25μl of either heat-inactivated (56°C waterbath for 30 minutes) or normal baby rabbit complement were added to each well. Immediately after the addition of the baby rabbit complement, 22μl of each sample/well were plated on Mueller-Hinton agar plates (time 0). The 96-well plate was incubated for 1 hour at 37°C with rotation and then 22μl of each sample/well were plated on Mueller-Hinton agar plates (time 1). After overnight incubation the colonies corresponding to time 0 and time 1 hour were counted.

**Example 1**

The following partial DNA sequence was identified in *N.meningitidis* <SEQ ID 1>:

```
  1 ..GCGGAATATG TTCAGTTCTC TATAGATTTG TTCAGTGTGG GTAAATCGGG
 51   GGGCGGTATA CCTAAGGCTA AGCCTGTGTT TGATGCGAAA CCGAGATGGG
101   AGGTTGATAG GAAGCTTAAT AAATTGACAA CTCGTGAGCA GGTGGAGAAA
151   AATGTTCAGG AAACGAGAAG AAGGAGTCAG AGTAGTCAGT TTAAAGCCCA
201   TGCGCAACGA GAATGGGAAA ATAAAACAGG GTTAGATTTT AATCATTTTA
251   TAGGTGGTGA TATCAATAAA AAAGGCACAG TAACAGGAGG GCATAGTCTA
301   ACCCGTGGTG ATGTACGGGT GATACAACAA ACCTCGGCAC CTGATAAACA
351   TGGGGT.TTA TCAAGCGACA GTGGAAATTN A
```

This corresponds to the amino acid sequence <SEQ 2; ORF46>:

```
  1 ..AEYVQFSIDL FSVGKSGGGI PKAKPVFDAK PRWEVDRKLN KLTTREQVEK
 51   NVQETRRRSQ SSQFKAHAQR EWENKTGLDF NHFIGGDINK KGTVTGGHSL
101   TRGDVRVIQQ TSAPDKHGXL SSDSGNX
```

Further work revealed further partial nucleotide sequence <SEQ ID 3>:

```
  1 ..GCAGTGTGCC TnCCGATGCA TGCACACGCC TCAnATTTGG CAAACGATTC
 51   TTTTATCCGG CAGGTTCTCG ACCGTCAGCA TTTCGAACCC GACGGGAAAT
101   ACCACCTATT CGGCAGCAGG GGGGAACTTG CCGAGCGCCA GTCTCATATC
151   GGATTGGGAA AAATACAAAG CCATCAGTTG GGCAACCTGA TGATTCAACA
201   GGCGGCCATT AAAGGAAATA TCGGCTACAT TGTCCGCTTT TCCGATCACG
251   GGCACGAAGT CCATTCCCCs TTCGACAACC ATGCCTCACA TTCCGATTCT
301   GATGAAGCCG GTAGTCCCGT TGACGGATTT AGCCTTTACC GCATCCATTG
351   GGACGGATAC GAACACCATC CCGCCGACGG CTATGACGGG CCACAGGGCG
401   GCGGCTATCC CGCTCCCAAA GGCGCGAGGG ATATATACAG TTACGACATA

451   AAAGGCGTTG CCCAAAATAT CCGCCTCAAC CTGACCGACA ACCGCAGCAC
501   CGGACAACGG CTTGCCGACC GTTTCCACAA TGCCGGTAGT ATGCTGACGC
551   AAGGAGTAGG CGACGGATTC AAACGCGCCA CCCGATACAG CCCCGAGCTG
601   GACAGATCGG GCAATGCCGC CGAAGCCTTC AACGGCACTG CAGATATCGT
651   TAAAAACATC ATCGGCGCTG CAGGAGAAAT TGT
```

This corresponds to the amino acid sequence <SEQ ID 4; ORF46-1>:

```
  1 ..AVCLPMHAHA SXLANDSFIR QVLDRQHFEP DGKYHLFGSR GELAERQSHI
 51   GLGKIQSHQL GNLMIQQAAI KGNIGYIVRF SDHGHEVHSP FDNHASHSDS
101   DEAGSPVDGF SLYRIHWDGY EHHPADGYDG PQGGGYPAPK GARDIYSYDI
151   KGVAQNIRLN LTDNRSTGQR LADRFHNAGS MLTQGVGDGF KRATRYSPEL
201   DRSGNAAEAF NGTADIVKNI IGAAGEI
```

Computer analysis of this amino acid sequence gave the following results:

Homology with a predicted ORF from *N.gonorrhoeae*

ORF46 shows 98.2% identity over a 111aa overlap with a predicted ORF (ORF46ng) from *N. gonorrhoeae:*

```
orf46.pep                   AEYVQFSIDLFSVGKSGGGIPKAKPVFDAKPRWEVDRKLNKLTTR   45
                                          |||||||||||||||||||||||||||||
orf46ng    PKTGVPFDGKGFPNFEKHVKYDTKLDIQELSGGGIPKAKPVFDAKPRWEVDRKLNKLTTR  217

orf46.pep  EQVEKNVQETRRRSQSSQFKAHAQREWENKTGLDFNHFIGGDINKKGTVTGGHSLTRGDV  105
           ||||||||||||||||||||||||||||||||||||||||||||||||:|||||||||||
orf46ng    EQVEKNVQETRRRSQSSQFKAHAQREWENKTGLDFNHFIGGDINKKGAVTGGHSLTRGDV  277

orf46.pep  RVIQQTSAPDKHGXLSSDSGN  126
           ||||||||||||| |||||||
orf46ng    RVIQQTSAPDKHGVLSSDSGN  298
```

A partial ORF46ng nucleotide sequence is predicted to encode a protein having partial amino acid sequence

<SEQ ID 5>:

```
  1 ..RRLKHCCHAR LGSAFHRKQD GAHQRFGRYG ATQRLCRSSH PRLGSPKPQC
 51   RTRHRSRQQY LYGSHPHQRD WSCPGKIQLG RHHGTSCRAV ADXRDRICER
101   EIRRQRQXCR CRLGKIPSLS IPKYPLKLEQ RYGKENITSS TVPPSNGKNV
151   KLADQRHPKT GVPFDGKGFP NFEKHVKYDT KLDIQELSGG GIPKAKPVFD
201   AKPRWEVDRK LNKLTTREQV EKNVQETRRR SQSSQFKAHA QREWENKTGL
251   DFNHFIGGDI NKKGAVTGGH SLTRGDVRVI QQTSAPDKHG VLSSDSGN*
```

Further work revealed the complete gonococcal DNA sequence <SEQ ID 6>:

```
   1 TTGGGCATTT CCCGCAAAAT ATCCCTTATT CTGTCCATAC TGGCAGTGTG
  51 CCTGCCGATG CATGCACACG CCTCAGATTT GGcaAACGAT CCCTTTATCC
 101 GgCaggttcT CGaccGTCAG CATTTCGaac ccgacggGAa ATACCaCCTA
 151 TTcggCaGCA GGGGGGAGCT TgccnagcGC aacggccATa tcggattggG
 201 aaacaTAcaa Agccatcagt tGggccacct gatgattcaa caggcggccg
 251 ttgaaggaaA TAtcgGctac attgtccgct tttccgatca cgggcacaaa
 301 ttccattcgc ccttcGAcaa ccaTGCCTCA CATTCCGATT CTGACGAAGC
 351 CGGTAGTCCC GTTGACGGAT TCAGCCTTTA CCGCATCCAT TGGGACGGAT
 401 ACGAACACCA TCCCGCCGAC GGCTATGACG GGCCACAGGG CGGCGGCTAT
 451 CCCGCTCCCA AAGGCGCGAG GGATATATAC AGCTACGACA TAAAAGGCGT
 501 TGCCCAAAAT ATCCGCCTCA ACCTGACCGA CAACCGCAGC ACCGGACAAC
 551 GGCTTGCCGA CCGTTTCCAC AATGCCGGCG CTATGCTGAC GCAAGGAGTA
 601 GGCGACGGAT TCAAACGCGC CACCCGATAC AGCCCCGAGC TGGACAGATC
 651 GGGCAATGCc gccGAAGCCT TCAACGGCAC TGCAGATATC GTCAAAAACA
 701 TCATCGGCGC GGCAGGAGAA ATTGTCGGCG CAGGCGATGC CGTGCagGGT
 751 ATAAGCGAAG GCTCAAACAT TGCTGTCATG CACGGCTTGG GTCTGCTTTC
 801 CACCGAAAAC AAGATGGCGC GCATCAACGA TTTGGCAGAT ATGGCGCAAC
 851 TCAAAGACTA TGCCGCAGCA GCCATCCGCG ATTGGGCAGT CCAAAACCCC
 901 AATGCCGCAC AAGGCATAGA AGCCGTCAGC AATATCTTTA TGGCAGCCAT
 951 CCCCATCAAA GGGATTGGAG CTGTCCGGGG AAAATACGGC TTGGGCGGCA
1001 TCACGGCACA TCCTGTCAAG CGGTCGCAGA TGGGCGCGAT CGCATTGCCG
1051 AAAGGGAAAT CCGCCGTCAG CGACAATTTT GCCGATGCGG CATACGCCAA
1101 ATACCCGTCC CCTTACCATT CCCGAAATAT CCGTTCAAAC TTGGAGCAGC
```

```
1151 GTTACGGCAA AGAAAACATC ACCTCCTCAA CCGTGCCGCC GTCAAACGGC
1201 AAAAATGTCA AACTGGCAGA CCAACGCCAC CCGAAGACAG GCGTACCGTT
1251 TGACGGTAAA GGGTTTCCGA ATTTTGAGAA GCACGTGAAA TATGATACGA
1301 AGCTCGATAT TCAAGAATTA TCGGGGGGCG GTATACCTAA GGCTAAGCCT
1351 GTGTTTGATG CGAAACCGAG ATGGGAGGTT GATAGGAAGC TTAATAAATT
1401 GACAACTCGT GAGCAGGTGG AGAAAAATGT TCAGGAAACG AGAAGAAGGA
1451 GTCAGAGTAG TCAGTTTAAA GCCCATGCGC AACGAGAATG GGAAAATAAA
1501 ACAGGGTTAG ATTTTAATCA TTTTATAGGT GGTGATATCA ATAAGAAAGG
1551 CACAGTAACA GGAGGGCATA GTCTAACCCG TGGTGATGTA CGGGTGATAC
1601 AACAAACCTC GGCACCTGAT AAACATGGGG TTTATCAAGC GACAGTGGAA
1651 ATTAAAAAGC CTGATGGAAG TTGGGAGGTG AAAACGAAAA AAGGTGGGAA
1701 AGTGATGACC AAGCACACCA TGTTCCCAAA AGATTGGGAT GAGGCTAGAA
1751 TTAGGGCTGA AGTTACTTCG GCTTGGGAAA GTAGAATAAT GCTTAAGGAT
1801 AATAAATGGC AGGGTACAAG TAAATCGGGT ATTAAAATAG AAGGATTTAC
1851 CGAACCTAAT AGAACAGCAT ATCCCATTTA TGAATAG
```

This corresponds to the amino acid sequence <SEQ ID 7 ; ORF46ng-1>:

```
  1  LGISRKISLI LSILAVCLPM HAHASDLAND PFIRQVLDRQ HFEPDGKYHL
 51  FGSRGELAXR NGHIGLGNIQ SHQLGHLMIQ QAAVEGNIGY IVRFSDHGHK
101  FHSPFDNHAS HSDSDEAGSP VDGFSLYRIH WDGYEHHPAD GYDGPQGGGY
151  PAPKGARDIY SYDIKGVAQN IRLNLTDNRS TGQRLADRFH NAGAMLTQGV
201  GDGFKRATRY SPELDRSGNA AEAFNGTADI VKNIIGAAGE IVGAGDAVQG
251  ISEGSNIAVM HGLGLLSTEN KMARINDLAD MAQLKDYAAA AIRDWAVQNP
301  NAAQGIEAVS NIFMAAIPIK GIGAVRGKYG LGGITAHPVK RSQMGAIALP
351  KGKSAVSDNF ADAAYAKYPS PYHSRNIRSN LEQRYGKENI TSSTVPPSNG
401  KNVKLADQRH PKTGVPFDGK GFPNFEKHVK YDTKLDIQEL SGGGIPKAKP
451  VFDAKPRWEV DRKLNKLTTR EQVEKNVQET RRRSQSSQFK AHAQREWENK
501  TGLDFNHFIG GDINKKGTVT GGHSLTRGDV RVIQQTSAPD KHGVYQATVE
551  IKKPDGSWEV KTKKGGKVMT KHTMFPKDWD EARIRAEVTS AWESRIMLKD
601  NKWQGTSKSG IKIEGFTEPN RTAYPIYE*
```

ORF46ng-1 and ORF46-1 show 94.7% identity in 227 aa overlap:

```
                                     10        20        30        40
orf46-1.pep                  AVCLPMHAHASXLANDSFIRQVLDRQHFEPDGKYHLFGSRGELAER
                             ||||||||||| |||| ||||||||||||||||||||||||||| |
orf46ng-1      LGISRKISLILSILAVCLPMHAHASDLANDPFIRQVLDRQHFEPDGKYHLFGSRGELAXR
                       10        20        30        40        50        60

                 50        60        70        80        90       100
orf46-1.pep    QSHIGLGKIQSHQLGNLMIQQAAIKGNIGYIVRFSDHGHEVHSPFDNHASHSDSDEAGSP
               ::|||||:|||||||:|||||||::||||||||||||||: |||||||||||||||||||
orf46ng-1      NGHIGLGNIQSHQLGHLMIQQAAVEGNIGYIVRFSDHGHKFHSPFDNHASHSDSDEAGSP
                       70        80        90       100       110       120

                110       120       130       140       150       160
orf46-1.pep    VDGFSLYRIHWDGYEHHPADGYDGPQGGGYPAPKGARDIYSYDIKGVAQNIRLNLTDNRS
               ||||||||||||||||||||||||||||||||||||||||||||||||||||||||||||
orf46ng-1      VDGFSLYRIHWDGYEHHPADGYDGPQGGGYPAPKGARDIYSYDIKGVAQNIRLNLTDNRS
                      130       140       150       160       170       180

                170       180       190       200       210       220
orf46-1.pep    TGQRLADRFHNAGSMLTQGVGDGFKRATRYSPELDRSGNAAEAFNGTADIVKNIIGAAGE
               |||||||||||||:||||||||||||||||||||||||||||||||||||||||||||||
orf46ng-1      TGQRLADRFHNAGAMLTQGVGDGFKRATRYSPELDRSGNAAEAFNGTADIVKNIIGAAGE
                      190       200       210       220       230       240

orf46-1.pep    I
               |
orf46ng-1      IVGAGDAVQGISEGSNIAVMHGLGLLSTENKMARINDLADMAQLKDYAAAAIRDWAVQNP
                      250       260       270       280       290       300
```

Homology with a predicted ORF *from N.meningitidis* (strain A)

ORF46ng- shows 87.4% identity over a 486aa overlap with an ORF (ORF46a) from strain A of *N. meningitidis:*

```
                       10        20        30        40        50        60
```

```
orf46a.pep     LGISRKISLILSILAVCLPMHAHASDLANDSFIRQVLDRQHFEPDGKYHLFGSRGELAER
               |||||||||||||||||||||||||||||| ||||||||||||||||||||||||||| |
orf46ng-1      LGISRKISLILSILAVCLPMHAHASDLANDPFIRQVLDRQHFEPDGKYHLFGSRGELAXR
                        10        20        30        40        50        60

                        70        80        90       100       110       120
orf46a.pep     SGHIGLGNIQSHQLGNLFIQQAAIKGNIGYIVRFSDHGHEVHSPFDNHASHSDSDEAGSP
               :||||||||||||||:|:|||||::||||||||||||||: |||||||||||||||||||
orf46ng-1      NGHIGLGNIQSHQLGHLMIQQAAVEGNIGYIVRFSDHGHKFHSPFDNHASHSDSDEAGSP
                        70        80        90       100       110       120

                       130       140       150       160       170       180
orf46a.pep     VDGFSLYRIHWDGYEHHPADGYDGPQGGGYPAPKGARDIYSYDIKGVAQNIRLNLTDNRS
               ||||||||||||||||||||||||||||||||||||||||||||||||||||||||||||
orf46ng-1      VDGFSLYRIHWDGYEHHPADGYDGPQGGGYPAPKGARDIYSYDIKGVAQNIRLNLTDNRS
                       130       140       150       160       170       180

                       190       200       210       220       230       240
orf46a.pep     TGQRLVDRFHNTGSMLTQGVGDGFKRATRYSPELDRSGNAAEAFNGTADIVKNIIGAAGE
               |||||:|||||:|:||||||||||||||||||||||||||||||||||||||||||||||
orf46ng-1      TGQRLADRFHNAGAMLTQGVGDGFKRATRYSPELDRSGNAAEAFNGTADIVKNIIGAAGE
                       190       200       210       220       230       240

                       250       260       270       280       290       300
orf46a.pep     IVGAGDAVQGISEGSNIAVMHGLGLLSTENKMARINDLADMAQLKDYAAAAIRDWAVQNP
               ||||||||||||||||||||||||||||||||||||||||||||||||||||||||||||
orf46ng-1      IVGAGDAVQGISEGSNIAVMHGLGLLSTENKMARINDLADMAQLKDYAAAAIRDWAVQNP
                       250       260       270       280       290       300

                       310       320       330       340       350       360
orf46a.pep     NAAQGIEAVSNIFTAVIPVKGIGAVRGKYGLGGITAHPVKRSQMGEIALPKGKSAVSDNF
               ||||||||||||| |:||:|||||||||||||||||||||||||| ||||||||||||||
orf46ng-1      NAAQGIEAVSNIFMAAIPIKGIGAVRGKYGLGGITAHPVKRSQMGAIALPKGKSAVSDNF
                       310       320       330       340       350       360

                       370       380       390       400       410       420
orf46a.pep     ADAAYAKYPSPYHSRNIRSNLEQRYGKENITSSTVPPSNGKNVKLANKRHPKTKVPFDGK
               ||||||||||||||||||||||||||||||||||||||||||||||::||||| ||||||
orf46ng-1      ADAAYAKYPSPYHSRNIRSNLEQRYGKENITSSTVPPSNGKNVKLADQRHPKTGVPFDGK
                       370       380       390       400       410       420

                       430       440           450         460       470
orf46a.pep     GFPNFEKDVKYDTRINTAVPQVN----PIDEPVFN--PKGSVGSAHSWSITARIQYAKLP
               ||||||| |||||:::  : :::    |  :|||:  |:  |    : ::|:| |  |
orf46ng-1      GFPNFEKHVKYDTKLD--IQELSGGGIPKAKPVFDAKPRWEVDRKLN-KLTTREQVEKNV
                        430         440       450       460       470

                     480       490       500       510       520       530
orf46a.pep     RQGRIRYIPPKNYSPSAPLPKGPNNGYLDKFGNEWTKGPSRTKGQEFEWDVQLSKTGREQ
               :: | |
orf46ng-1      QETRRRSQSSQFKAHAQREWENKTGLDFNHFIGGDINKKGTVTGGHSLTRGDVRVIQQTS
               480       490       500       510       520       530
```

The complete length ORF46a DNA sequence <SEQ ID 8> is:

```
   1  TTGGGCATTT CCCGCAAAAT ATCCCTTATT CTGTCCATAC TGGCAGTGTG
  51  CCTGCCGATG CATGCACACG CCTCAGATTT GGCAAACGAT TCTTTTATCC
 101  GGCAGGTTCT CGACCGTCAG CATTTCGAAC CCGACGGGAA ATACCACCTA
 151  TTCGGCAGCA GGGGGGAACT TGCCGAGCGC AGCGGTCATA TCGGATTGGG
 201  AAACATACAA AGCCATCAGT TGGGCAACCT GTTCATCCAG CAGGCGGCCA
 251  TTAAAGGAAA TATCGGCTAC ATTGTCCGCT TTTCCGATCA CGGGCACGAA
 301  GTCCATTCCC CCTTCGACAA CCATGCCTCA CATTCCGATT CTGATGAAGC
 351  CGGTAGTCCC GTTGACGGAT TCAGCCTTTA CCGCATCCAT TGGGACGGAT
 401  ACGAACACCA TCCCGCCGAC GGCTATGACG GGCCACAGGG CGGCGGCTAT
 451  CCCGCTCCCA AAGGCGCGAG GGATATATAC AGCTACGACA TAAAAGGCGT
 501  TGCCCAAAAT ATCCGCCTCA ACCTGACCGA CAACCGCAGC ACCGGACAAC
 551  GGCTTGTCGA CCGTTTCCAC AATACCGGTA GTATGCTGAC GCAAGGAGTA
 601  GGCGACGGAT TCAAACGCGC CACCCGATAC AGCCCCGAGC TGGACAGATC
 651  GGGCAATGCC GCCGAAGCTT TCAACGGCAC TGCAGATATC GTCAAAAACA
 701  TCATCGGCGC GGCAGGAGAA ATTGTCGGCG CAGGCGATGC CGTGCAGGGT
 751  ATAAGCGAAG GCTCAAACAT TGCTGTTATG CACGGCTTGG GTCTGCTTTC
 801  CACCGAAAAC AAGATGGCGC GCATCAACGA TTTGGCAGAT ATGGCGCAAC
```

```
 851  TCAAAGACTA TGCCGCAGCA GCCATCCGCG ATTGGGCAGT CCAAAACCCC
 901  AATGCCGCAC AAGGCATAGA AGCCGTCAGC AATATCTTTA CGGCAGTCAT
 951  CCCCGTCAAA GGGATTGGAG CTGTTCGGGG AAAATACGGC TTGGGCGGCA
1001  TCACGGCACA TCCTGTCAAG CGGTCGCAGA TGGGCGAGAT CGCATTGCCG
1051  AAAGGGAAAT CCGCCGTCAG CGACAATTTT GCCGATGCGG CATACGCCAA
1101  ATACCCGTCC CCTTACCATT CCCGAAATAT CCGTTCAAAC TTGGAGCAGC
1151  GTTACGGCAA AGAAAACATC ACCTCCTCAA CCGTGCCGCC GTCAAACGGA
1201  AAGAATGTGA AACTGGCAAA CAAACGCCAC CCGAAGACCA AAGTGCCGTT
1251  TGACGGTAAA GGGTTTCCGA ATTTTGAAAA AGACGTAAAA TACGATACGA
1301  GAATTAATAC CGCTGTACCA CAAGTGAATC CTATAGATGA ACCCGTCTTT
1351  AATCCTAAAG GTTCTGTCGG ATCGGCTCAT TCTTGGTCTA TAACTGCCAG
1401  AATTCAATAC GCAAAATTAC CAAGGCAAGG TAGAATCAGA TATATCCCAC
1451  CTAAAAATTA CTCTCCTTCA GCACCGCTAC CAAAAGGACC TAATAATGGA
1501  TATTTGGATA AATTTGGTAA TGAATGGACT AAAGGTCCAT CAAGAACTAA
1551  AGGTCAAGAA TTTGAATGGG ATGTTCAATT GTCTAAAACA GGAAGAGAGC
1601  AACTTGGATG GGCTAGTAGG GATGGTAAGC ATTTAAATAT ATCAATTGAT
1651  GGAAAGATTA CACACAAATG A
```

This corresponds to the amino acid sequence <SEQ ID 9>:

```
   1  LGISRKISLI LSILAVCLPM HAHASDLAND SFIRQVLDRQ HFEPDGKYHL
  51  FGSRGELAER SGHIGLGNIQ SHQLGNLFIQ QAAIKGNIGY IVRFSDHGHE
 101  VHSPFDNHAS HSDSDEAGSP VDGFSLYRIH WDGYEHHPAD GYDGPQGGGY
 151  PAPKGARDIY SYDIKGVAQN IRLNLTDNRS TGQRLVDRFH NTGSMLTQGV
 201  GDGFKRATRY SPELDRSGNA AEAFNGTADI VKNIIGAAGE IVGAGDAVQG
 251  ISEGSNIAVM HGLGLLSTEN KMARINDLAD MAQLKDYAAA AIRDWAVQNP
 301  NAAQGIEAVS NIFTAVIPVK GIGAVRGKYG LGGITAHPVK RSQMGEIALP
 351  KGKSAVSDNF ADAAYAKYPS PYHSRNIRSN LEQRYGKENI TSSTVPPSNG
 401  KNVKLANKRH PKTKVPFDGK GFPNFEKDVK YDTRINTAVP QVNPIDEPVF
 451  NPKGSVGSAH SWSITARIQY AKLPRQGRIR YIPPKNYSPS APLPKGPNNG
 501  YLDKFGNEWT KGPSRTKGQE FEWDVQLSKT GREQLGWASR DGKHLNISID
 551  GKITHK*
```

Based on this analysis, including the presence of a RGD sequence in the gonococcal protein, typical of adhesins, it is predicted that the proteins from *N.meningitidis* and *N.gonorrhoeae,* and their epitopes, could be useful antigens for vaccines or diagnostics, or for raising antibodies.

It will be appreciated that the invention has been described by means of example only, and that modifications may be made whilst remaining within the scope of the invention.

## Claims

1. A protein comprising an amino acid sequence selected from the group consisting of SEQ IDs 2, 4, 5, 7, and 9 presented in Example 1.

2. A protein having 80% or greater sequence identity to an amino acid sequence selected from the group consisting of SEQ IDs 2, 4, 5, 7, or 9.

3. A protein comprising a fragment of an amino acid sequence selected from the group consisting of SEQ IDs 2, 4, 5, 7, and 9, wherein the fragment comprises at least twenty consecutive amino acids and includes an epitope of said SEQ ID.

4. A nucleic acid molecule which encodes a protein according to any one of claims I to 3.

5. A nucleic acid molecule according to claim 4, comprising a nucleotide sequence selected from the group consisting of SEQ IDs 1, 3, 6 and 8.

6. A nucleic acid molecule comprising a nucleotide sequence complementary to a nucleic acid molecule according to any one of claims 4 or 5.

7. A composition comprising a protein, or a nucleic acid molecule according to any preceding claim.

8. A composition according to claim 7 being a vaccine composition or a diagnostic composition.

9. A composition according to claim 7 or claim 8 for use as a pharmaceutical.

10. The use of a composition according to claim 7 in the manufacture of a medicament for the treatment or prevention of infection due to Neisserial bacteria.

## Patentansprüche

1. Protein, das eine Aminosäuresequenz umfasst, die ausgewählt ist aus der Gruppe bestehend aus SEQ ID NO: 2, 4, 5, 7 und 9, die in Beispiel 1 gezeigt sind.

2. Protein, das 80% oder mehr Sequenzidentität zu einer Aminosäuresequenz aufweist, die ausgewählt ist aus der Gruppe bestehend aus SEQ ID NO: 2, 4, 5, 7 oder 9.

3. Protein, das ein Fragment einer Aminosäuresequenz umfasst, die ausgewählt ist aus der Gruppe bestehend aus SEQ ID NO: 2, 4, 5, 7 oder 9, wobei das Fragment mindestens zwanzig aufeinanderfolgende Aminosäuren und ein Epitop dieser SEQ ID NO umfasst.

4. Nukleinsäuremolekül, das für ein Protein nach einem der Ansprüche 1-3 kodiert.

5. Nukleinsäuremolekül nach Anspruch 4, das eine Nukleotidsequenz umfasst, die ausgewählt ist aus der Gruppe bestehend aus SEQ ID NO: 1, 3, 6 und 8.

6. Nukleinsäuremolekül, das eine Nukleotidsequenz umfasst, die komplementär zu einem Nukleinsäuremolekül nach einem der Ansprüche 4 oder 5 ist.

7. Zusammensetzung, die ein Protein oder ein Nukleinsäuremolekül nach einem der vorhergehenden Ansprüche umfasst.

8. Zusammensetzung nach Anspruch 7, die eine Impfstoff-Zusammensetzung oder eine diagnostische Zusammensetzung ist.

9. Zusammensetzung nach Anspruch 7 oder Anspruch 8 zur Verwendung als pharmazeutisches Mittel.

10. Verwendung einer Zusammensetzung nach Anspruch 7 zur Herstellung eines Medikaments zur Behandlung oder Vermeidung einer durch Neisseria-Bakterien verursachten Infektion.

## Revendications

1. Protéine comprenant une séquence d'acides aminés choisie dans le groupe constitué par SÉQ ID NO : 2, 4, 5, 7 et 9 présentées dans l'exemple 1.

2. Protéine présentant une identité de séquence de 80 % ou plus avec une séquence d'acides aminés choisie dans le groupe constitué par SÉQ ID NO : 2, 4, 5, 7 ou 9.

3. Protéine comprenant un fragment d'une séquence d'acides aminés choisie dans le groupe constitué par SÉQ ID NO : 2, 4, 5, 7 et 9, dans laquelle le fragment comprend au moins 20 acides aminés consécutifs et comprenant un épitope de ladite SÉQ ID.

4. Molécule d'acide nucléique qui code pour une protéine selon l'une quelconque des revendications 1 à 3.

5. Molécule d'acide nucléique selon la revendication 4, qui comprend une séquence de nucléotides choisie dans le groupe constitué par SÉQ ID NO : 1, 3, 6 et 8.

6. Molécule d'acide nucléique comprenant une séquence de nucléotides complémentaire d'une molécule d'acide nucléique selon l'une quelconque des revendications 4 ou 5.

7. Composition comprenant une protéine, ou une molécule d'acide nucléique selon l'une quelconque des revendications précédentes.

8. Composition selon la revendication 7, étant une composition vaccinale ou une composition de diagnostic.

9. Composition selon la revendication 7 ou la revendication 8, destinée à être utilisée en tant que produit pharmaceutique.

10. Utilisation d'une composition selon la revendication 7, dans la fabrication d'un médicament destiné au traitement ou à la prévention d'une infection par les bactéries Neisseria.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0467714 A
- WO 9629412 A
- WO 9711181 A
- US 5753235 A
- EP 127839 A
- EP 155476 A
- WO 89046699 A
- US 5693506 A
- US 5659122 A
- US 5608143 A
- US 4738921 A
- EP 0036776 A
- EP 0121775 A
- US 4689406 A
- US 4551433 A
- EP 0267851 A
- EP 0219237 A
- EP 0324647 A, Chey
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- EP 0284044 A
- EP 0329203 A
- US 4876197 A
- US 4880734 A
- EP 0164556 A
- EP 0196056 A
- WO 88024066 A
- EP 0012873 A
- JP O62096086 B
- US 4588684 A
- EP 0060057 A
- US 4546083 A
- US 4870008 A
- EP 0324274 A
- WO 8902463 A
- US 4837148 A
- US 4929555 A
- WO 9820734 A
- WO 9014837 A
- US 5591624 A
- WO 9637626 A
- WO 9530763 A
- WO 9205266 A
- GB 2200651 A
- EP 0415731 A
- EP 0345242 A
- EP 0334301 A
- WO 8902468 A
- WO 8905349 A
- WO 8909271 A
- WO 9002806 A
- WO 9007936 A
- WO 9403622 A
- WO 9325698 A
- WO 9325234 A
- WO 9311230 A
- WO 9310218 A
- WO 9102805 A
- WO 9102825 A
- WO 9507994 A
- US 5219740 A
- US 4405712 A
- US 4861719 A
- US 4980289 A
- US 4777127 A
- WO 9307283 A
- WO 9306223 A
- WO 9307282 A
- WO 9412649 A
- WO 9303769 A
- WO 9319191 A
- WO 9428938 A
- WO 9511984 A
- WO 9500655 A
- WO 9527071 A
- WO 9529993 A
- WO 9534671 A
- WO 9605320 A
- WO 9408026 A
- WO 9411506 A
- WO 9424299 A
- WO 9514102 A
- WO 9524297 A
- WO 9502697 A
- WO 9428152 A
- WO 9509241 A
- WO 9525807 A
- WO 9505835 A
- WO 9418922 A
- WO 9509654 A
- WO 9309239 A

- US 5478745 A
- US 4797368 A, Carter
- US 5139941 A, Muzyczka
- US 5474935 A, Chartejee
- WO 94288157 A, Kotin
- US 5354678 A
- US 5173414 A
- US 5252479 A
- US 5288641 A
- EP 0176170 A, Roizman
- WO 9504139 A
- WO 9009441 A
- WO 9207945 A
- EP 0453242 A
- US 5091309 A
- US 5217879 A
- WO 9210578 A
- US 08405627 B
- WO 9421792 A
- US SN08679640 A
- US 4603112 A
- US 4769330 A
- WO 8901973 A
- US 5166057 A
- EP 0386882 A
- EP 0440219 A
- US 08366787 B
- US 08240030 B
- US 08404796 B
- US 5149655 A
- US 5206152 A
- WO 9211033 A
- US 60023867 B
- WO 9011092 A
- US 5580859 A
- US 5422120 A
- WO 9513796 A
- WO 9423697 A
- WO 9114445 A
- EP 524968 A
- US SN60023867 P
- WO 921103 A
- US 4762915 A
- EP 0524968 A
- WO 9314778 A
- US 4683195 A
- US 4683202 A


**Non-patent literature cited in the description**


- Vaccines Against Gonococcal Infection. **Meitzner ; Cohen.** New Generation Vaccines. Marcel Dekker, 1997, 817-842
- **Lieberman et al.** Safety and Immunogenicity of a Serogroups A/C Neisseria meningitidis Oligosaccharide-Protein Conjugate Vaccine in Young Children. *JAMA,* 1996, vol. 275 (19), 1499-1503
- **Schuchat et al.** Bacterial Meningitis in the United States in 1995. *NEngl J Med,* 1997, vol. 337 (14), 970-976
- *Morbidity and Mortality weekly report,* 1997, vol. 46 (RR-5
- **Zollinger WD.** New and Improved Vaccines Against Meningococcal Disease. *New Generation Vaccines,* 469-488
- **Costantino et al.** Development and phase I clinical testing of a conjugate vaccine against meningococcus A and C. *Vaccine,* 1992, vol. 10, 691-698
- **Romero ; Outschoorn.** Current status of Meningococcal group B vaccine candidates: capsular or non-capsular?. *Clin Microbiol Rev,* 1994, vol. 7 (4), 559-575
- **Poolman JT.** Development of a meningococcal vaccine. *Infect. Agents Dis.,* 1992, vol. 4, 13-28
- **Ala'Aldeen ; Borriello.** The meningococcal transferrin-binding proteins 1 and 2 are both surface exposed and generate bactericidal antibodies capable of killing homologous and heterologous strains. *Vaccine,* 1996, vol. 14 (1), 49-53
- **Sambrook.** Molecular Cloning; A Laboratory Manual. 1989
- DNA Cloning. 1985, vol. I, ii
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **B. Perbal.** A Practical Guide to Molecular Cloning. 1984
- Methods in Enzymology series. Academic Press, Inc,
- Gene Transfer Vectors for Mammalian Cells. Cold Spring Harbor Laboratory, 1987, vol. 154, 155
- Immunochemical Methods in Cell and Molecular Biology. Academic Press, 1987
- **Scopes.** Protein Purification: Principles and Practice. Springer-Verlag, 1987
- Handbook of Experimental Immunology. 1986, vol. I-IV
- Expression of Cloned Genes in Mammalian Cells. **Sambrook et al.** Molecular Cloning: A Laboratory Manual. 1989
- **Maniatis et al.** *Science,* 1987, vol. 236, 1237
- **Alberts et al.** Molecular Biology of the Cell. 1989
- **Dijkema et al.** *EMBO J.,* 1985, vol. 4, 761
- **Gorman et al.** *Proc. Natl. Acad. Sci.,* 1982, vol. 79, 6777

- **Boshart et al.** *Cell,* 1985, vol. 41, 521
- **Sassone-Corsi ; Borelli.** *Trends Genet.,* 1986, vol. 2, 215
- **Birnstiel et al.** *Cell,* 1985, vol. 41, 349
- Termination and 3' end processing of eukaryotic RNA. **Proudfoot ; Whitelaw.** Transcription and splicing. 1988
- **Proudfoot.** *Trends Biochem. Sci.,* 1989, vol. 14, 105
- Expression of cloned genes in cultured mammalian cells. **Sambrook et al.** Molecular Cloning: A Laboratory Manual. 1989
- **Gluzman.** *Cell,* 1981, vol. 23, 175
- **Kaufman et al.** *Mol. Cell. Biol.,* 1989, vol. 9, 946
- **Shimizu et al.** *Mol. Cell. Biol.,* 1986, vol. 6, 1074
- **Luckow ; Summers.** *Virology,* 1989, vol. 17, 31
- **Miller et al.** *Ann. Rev. Microbiol.,* 1988, vol. 42, 177
- The Regulation of Baculovirus Gene Expression. **Friesen et al.** The Molecular Biology of Baculoviruses. 1986
- **Vlak et al.** *J. Gen. Virol.,* 1988, vol. 69, 765
- **Maeda et al.** *Nature,* 1985, vol. 315, 592
- **Lebacq-Verheyden et al.** *Molec. Cell. Biol.,* 1988, vol. 8, 3129
- **Smith et al.** *Proc. Nat'l Acad. Sci. USA,* 1985, vol. 82, 8404
- **Miyajima et al.** *Gene,* 1987, vol. 58, 273
- **Martin et al.** *DNA,* 1988, vol. 7, 99
- **Smith et al.** *Mol. Cell. Biol.,* 1983, vol. 3, 2156
- **Miller et al.** *Bioessays,* 1989, vol. 4, 91
- Current Protocols in Microbiology. 1990, vol. 2
- **Carbonell et al.** *J. Virol.,* 1985, vol. 56, 153
- **Wright.** *Nature,* 1986, vol. 321, 718
- **Fraser et al.** *Vitro Cell. Dev. Biol.,* 1989, vol. 25, 225
- **Zenk.** *Phytochemistry,* 1991, vol. 30, 3861-3863
- **Vaulcombe et al.** *Mol. Gen. Genet.,* 1987, vol. 209, 33-40
- **Chandler et al.** *Plant Molecular Biology,* 1984, vol. 3, 407-418
- **Rogers.** *J. Biol. Chem.,* 1985, vol. 260, 3731-3738
- **Rothstein et al.** *Gene,* 1987, vol. 55, 353-356
- **Whittier et al.** *Nucleic Acids Research,* 1987, vol. 15, 2515-2535
- **Wirsel et al.** *Molecular Microbiology,* 1989, vol. 3, 3-14
- **Yu et al.** *Gene,* 1992, vol. 122, 247-253
- **R.L. Jones ; J. MacMillin.** Gibberellins: in: Advanced Plant Physiology. Pitman Publishing Limited, 1984, 21-52
- **Sheen.** *Plant Cell,* 1990, vol. 2, 1027-1038
- **Maas et al.** *EMBO J.,* 1990, vol. 9, 3447-3452
- **Benkel ; Hickey.** *Proc. Natl. Acad. Sci.,* 1987, vol. 84, 1337-1339
- **Wilmink ; Dons.** *Plant Mol. Biol. Reptr,* 1993, vol. 11 (2), 165-185
- **Reed ; Maniatis.** *Cell,* 1985, vol. 41, 95-105
- **Crossway.** *Mol. Gen. Genet,* 1985, vol. 202, 179-185
- **Krens et al.** *Nature,* 1982, vol. 296, 72-74
- **Klein et al.** *Nature,* 1987, vol. 327, 70-73
- **Knudsen ; Muller.** *Planta,* 1991, vol. 185, 330-336
- **Fraley et al.** *Proc. Natl. Acad. Sci. USA,* 1982, vol. 79, 1859-1863
- **Fromm et al.** *Proc. Natl Acad. Sci. USA,* 1985, vol. 82, 5824
- **Raibaud et al.** *Annu. Rev. Genet.,* 1984, vol. 18, 173
- **Chang et al.** *Nature,* 1977, vol. 198, 1056
- **Goeddel et al.** *Nuc. Acids Res.,* 1980, vol. 8, 4057
- **Yelverton et al.** *Nucl. Acids Res.,* 1981, vol. 9, 731
- The cloning of interferon and other mistakes. **Weissmann.** Interferon. 1981
- **Shimatake et al.** *Nature,* vol. 292, 128
- **Amann et al.** *Gene,* 1983, vol. 25, 167
- **de Boer et al.** *Proc. Natl. Acad. Sci.,* 1983, vol. 80, 21
- **Studier et al.** *J. Mol. Biol.,* 1986, vol. 189, 113
- **Tabor et al.** *Proc Natl. Acad. Sci.,* 1985, vol. 82, 1074
- Genetic signals and nucleotide sequences in messenger RNA. **Steitz et al.** Biological Regulation and Development: Gene Expression. 1979
- Expression of cloned genes in Escherichia coli. **Sambrook et al.** Molecular Cloning: A Laboratory Manual. 1989
- **Nagai et al.** *Nature,* 1984, vol. 309, 810
- **Jia et al.** *Gene,* 1987, vol. 60, 197
- **Allen et al.** *J. Biotechnol.,* 1987, vol. 5, 93
- **Makoff et al.** *J. Gen. Microbiol.,* 1989, vol. 135, 11
- **Miller et al.** *Bio/Technology,* 1989, vol. 7, 698
- **Masui et al.** *Experimental Manipulation of Gene Expression,* 1983
- **Ghrayeb et al.** *EMBO J.,* 1984, vol. 3, 2437
- **Oka et al.** *Proc. Natl. Acad. Sci.,* 1985, vol. 82, 7212
- **Palva et al.** *Proc. Natl. Acad. Sci. USA,* 1982, vol. 79, 5582
- **Davies et al.** *Annu. Rev. Microbiol.,* 1978, vol. 32, 469
- **Shimatake et al.** *Nature,* 1981, vol. 292, 128
- **Amann et al.** *Gene,* 1985, vol. 40, 183
- **Powell et al.** *Appl. Environ. Microbiol.,* 1988, vol. 54, 655

- **Masson et al.** *FEMS Microbiol. Lett.,* 1989, vol. 60, 273
- **Miller et al.** *Proc. Natl. Acad. Sci.,* 1988, vol. 85, 856
- **Wang et al.** *J. Bacteriol.,* 1990, vol. 172, 949
- **Cohen et al.** *Proc. Natl. Acad. Sci.,* 1973, vol. 69, 2110
- **Dower et al.** *Nucleic Acids Res.,* 1988, vol. 16, 6127
- An improved method for transformation of Escherichia coli with ColE1-derived plasmids. **Kushner.** Genetic Engineering: Proceedings of the International Symposium on Genetic Engineering. 1978
- **Mandel et al.** *J. Mol. Biol.,* 1970, vol. 53, 159
- **Taketo.** *Biochim. Biophys. Acta,* 1988, vol. 949, 318
- **Chassy et al.** *FEMS Microbio/. Lett.,* 1987, vol. 44, 173
- **Fiedler et al.** *Anal. Biochem,* 1988, vol. 170, 38
- **Augustin et al.** *FEMS Microbiol. Lett.,* 1990, vol. 66, 203
- **Barany et al.** *J. Bacteriol.,* 1980, vol. 144, 698
- Transformation of Streptococcus lactis by electroporation. **Harlander.** Streptococcal Genetics. 1987
- **Perry et al.** *Infect. Immun.,* 1981, vol. 32, 1295
- **Somkuti et al.** *Proc. 4th Evr. Cong. Biotechnology,* 1987, vol. 1, 412
- **Myanohara et al.** *Proc. Natl. Acad. Sci. USA,* 1983, vol. 80, 1
- **Cohen et al.** *Proc. Natl. Acad. Sci. USA,* 1980, vol. 77, 1078
- **Henikoff et al.** *Nature,* 1981, vol. 283, 835
- **Hollenberg et al.** *Curr. Topics Microbiol. Immunol.,* 1981, vol. 96, 119
- The Expression of Bacterial Antibiotic Resistance Genes in the Yeast Saccharomyces cerevisiae. **Hollenberg et al.** Plasmids of Medical, Environmental and Commercial Importance. 1979
- **Mercerau-Puigalon et al.** *Gene,* 1980, vol. 11, 163
- **Panthier et al.** *Curr. Genet.,* 1980, vol. 2, 109
- **Botstein et al.** *Gene,* 1979, vol. 8, 17-24
- **Brake et al.** *Proc. Natl. Acad. Sci USA,* 1984, vol. 81, 4642-4646
- **Stinchcomb et al.** *J. Mol. Biol.,* 1982, vol. 158, 157
- **Orr-Weaver et al.** *Methods in Enzymol.,* 1983, vol. 101, 228-245
- **Rine et al.** *Proc. Natl. Acad. Sci. USA,* 1983, vol. 80, 6750
- **Butt et al.** *Microbiol, Rev.,* 1987, vol. 51, 351
- **Kurtz et al.** *Mol. Cell. Biol.,* 1986, vol. 6, 142
- **Kunze et al.** *J. Basic Microbiol.,* 1985, vol. 25, 141
- **Gleeson et al.** *J. Gen. Microbiol.,* 1986, vol. 132, 3459
- **Roggenkamp et al.** *Mol. Gen. Genet.,* 1986, vol. 202, 302
- **Das et al.** *J. Bacteriol.,* 1984, vol. 158, 1165
- **De Louvencourt et al.** *J. Bacteriol.,* 1983, vol. 154, 737
- **Van den Berg et al.** *Bio/Technology,* 1990, vol. 8, 135
- **Cregg et al.** *Mol. Cell. Biol.,* 1985, vol. 5, 3376
- **Hinnen et al.** *Proc. Natl. Acad. Sci. USA,* 1978, vol. 75, 1929
- **Ito et al.** *J. Bacteriol.,* 1983, vol. 153, 163
- **Beach ; Nurse.** *Nature,* 1981, vol. 300, 706
- **Davidow et al.** *Curr. Genet.,* 1985, vol. 10, 380471
- **Gaillardin et al.** *Curr. Genet.,* 1985, vol. 10, 49
- **Kunze et al.** *J. Basic Microbiol.,* 1985, vol. 25, 141, J. Basic Microbiol.
- **De Louvencourt et al.** *J. Bacteriol.,* 1983, vol. 154, 1165
- **Van den Berg et al.** *BiolTechnology,* 1990, vol. 8, 135
- **Davidow et al.** *Curr. Genet.,* 1985, vol. 10, 39
- Remington's Pharmaceutical Sciences. Mack Pub. Co, 1991
- **Robinson ; Torres.** *Seminars in Immunology,* 1997, vol. 9, 271-283
- **Donnelly et al.** *Annu Rev Immunol,* 1997, vol. 15, 617-648
- **Jolly.** *Cancer Gene Therapy,* 1994, vol. 1, 51-64
- **Kimura.** *Human Gene Therapy,* 1994, vol. 5, 845-852
- **Connelly.** *Human Gene Therapy,* 1995, vol. 6, 185-193
- **Kaplitt.** *Nature Genetics,* 1994, vol. 6, 148-153
- **ONeill.** *J. Viral.,* 1985, vol. 53, 160
- **Kelly.** *J. Virol.,* 1983, vol. 45, 291
- RNA Tumor Viruses. Cold Spring Harbor Laboratory, 1985
- **Hartle ; Rowe.** *J Virol,* 1976, vol. 19, 19-25
- **Vile.** *Cancer Res,* 1993, vol. 53, 3860-3864
- **Vile.** *Cancer Res,* vol. 53, 962-967
- **Ram.** *Cancer Res,* 1933, vol. 53, 83-88
- **Takamiya.** *J Neurosci Res,* 1992, vol. 33, 493-503
- **Baba.** *J Neurosurg,* 1993, vol. 79, 729-735
- **Mann.** *Cell,* 1983, vol. 33, 153
- **Cane.** *Proc Natl Acad Sci,* 1984, vol. 81, 6349
- **Miller.** *Human Gene Therapy,* 1990, 1
- **Berkner.** *Biotechniques,* 1988, vol. 6, 616

- **Rosenfeld.** *Science,* 1991, vol. 252, 431
- **Curiel.** *Hum. Gene Ther.,* 1992, vol. 3, 147-154
- **Nahreini.** *Gene,* 1993, vol. 124, 257-262
- **Samulski.** *J. Virol.,* 1987, vol. 61, 3096
- **Su.** *Human Gene Therapy,* 1996, vol. 7, 463-470
- **Geller.** *Science,* 1988, vol. 241, 1667-1669
- **Fink.** *Human Gene Therapy,* 1992, vol. 3, 11-19
- **Evans.** *Nature,* 1989, vol. 339, 385
- **Sabin.** *J. Biol. Standardization,* 1973, vol. 1, 115
- **Arnold.** *J Cell Biochem,* 1990, L401
- **Fisher-Hoch.** *Proc Natl Acad Sci,* 1989, vol. 86, 317
- **Flexner.** *Ann NY Acad Sci,* 1989, vol. 569, 86
- **Flexner.** *Vaccine,* 1990, vol. 8, 17
- **Mulligan.** *Nature,* 1979, vol. 277, 108
- **Madzak.** *J Gen Virol,* 1992, vol. 73, 1533
- **Enami.** *Proc Natl Acad Sci,* 1990, vol. 87, 3802-3805
- **Enami ; Palese.** *J Virol,* 1991, vol. 65, 2711-2713
- **Luytjes.** *Cell,* 1989, vol. 59, 110
- **McMichael.** *NEJ Med,* 1983, vol. 309, 13
- **Yap.** *Nature,* 1978, vol. 273, 238
- *Nature,* 1979, vol. 277, 108
- **Buchschacher.** *J. Viol,* 1992, vol. 66, 2731
- **Hamre.** *Proc Soc Exp Biol Med,* 1966, vol. 121, 190
- **Curiel.** *Hum Gene Ther,* 1992, vol. 3, 147-154
- **Wu.** *J Biol Chem,* 1989, vol. 264, 16985-16987
- **Philip.** *Mol Cell Biol,* 1994, vol. 14, 2411-2418
- **Woffendin.** *Proc Natl Acad Sci,* 1994, vol. 91, 1581-1585
- **Wu ; Wu.** *J. Biol. Chem.,* 1987, vol. 262, 4429-4432
- **Hucked.** *Biochem Pharmacol,* 1990, vol. 40, 253-263
- **Plank.** *Bioconjugate Chem,* 1992, vol. 3, 533-539
- **Woffendin et al.** *Proc. Natl. Acad. Sci. USA,* 1994, vol. 91 (24), 11581-11585
- **Stryer.** Biochemistry. W.H. Freeman, 1975, 236-240
- **Szoka.** *Biochem Biophys Acta,* 1980, vol. 600, 1
- **Bayer.** *Biochem Biophys Acta,* 1979, vol. 550, 464
- **Rivnay.** *Meth Enzymol,* 1987, vol. 149, 119
- **Wang.** *Proc Natl Acad Sci,* 1987, vol. 84, 7851
- **Plant.** *Anal Biochem,* 1989, vol. 176, 420
- **Hug ; Sleight.** *Biochim. Biophys. Acta,* 1991, vol. 1097, 1-17
- **Straubinger.** *Meth. Enzymol.,* 1983, vol. 101, 512-527
- **Felgner.** *Proc. Natl. Acad. Sci. USA,* 1987, vol. 84, 7413-7416
- **Malone.** *Proc. Natl. Acad. Sci. USA,* 1989, vol. 86, 6077-6081
- **Debs.** *J. Biol. Chem.,* 1990, vol. 265, 10189-10192
- **Szoka.** *Proc. Natl. Acad. Sci. USA,* 1978, vol. 75, 4194-4198
- **Straubinger.** *Meth. Immunol.,* 1983, vol. 101, 512-527
- **Papahadjopoulos.** *Biochim. Biophys. Acta,* 1975, vol. 394, 483
- **Wilson.** *Cell,* 1979, vol. 17, 77
- **Deamer ; Bangham.** *Biochim. Biophys. Acta,* 1976, vol. 443, 629
- **Ostro.** *Biochem. Biophys. Res. Commun.,* 1977, vol. 76, 836
- **Fraley.** *Proc. Natl. Acad. Sci. USA,* 1979, vol. 76, 3348
- **Enoch ; Strittmatter.** *Proc. Natl. Acad Sci. USA,* 1979, vol. 76, 145
- **Fraley.** *J. Biol. Chem.,* 1980, vol. 255, 10431
- **Szoka ; Papahadjopoulos.** *Proc. Natl. Acad. Sci. USA,* 1978, vol. 75, 145
- **Schaefer-Ridder.** *Science,* 1982, vol. 215, 166
- **Breslow.** *Annu Rev. Biochem,* 1985, vol. 54, 699
- **Law.** *Adv. Exp Med. Biol.,* 1986, vol. 151, 162
- **Chen.** *J Biol Chem,* 1986, vol. 261, 12918
- **Kane.** *Proc Natl Acad Sci USA,* 1980, vol. 77, 2465
- **Utermann.** *Hum Genet,* 1984, vol. 65, 232
- *Meth. Enzymol.,* 1986, 128
- **Pitas.** *J. Biochem.,* 1980, vol. 255, 5454-5460
- **Mahey.** *J Clin. Invest,* 1979, vol. 64, 743-750
- **Atkinson.** *Annu Rev Biophys Chem,* 1986, vol. 15, 403
- **Radding.** *Biochim Biophys Acta,* 1958, vol. 30, 443
- **Meinkoth ; Wahl.** *Anal. Biochem.,* 1984, vol. 138, 267-284
- **Matteucci et al.** *J. Am. Chem. Soc.,* 1981, vol. 103, 3185
- **Urdea et al.** *Proc. Natl. Acad. Sci. USA,* 1983, vol. 80, 7461
- **Agrawal ; Iyer.** *Curr Opin Biotechnol,* 1995, vol. 6, 12-19
- **Agrawal.** *TIBTECH,* 1996, vol. 14, 376-387
- **Corey.** *TIBTECH,* 1997, vol. 15, 224-229
- **Buchardt et al.** *TIBTECH,* 1993, vol. 11, 384-386
- **Mullis et al.** *Meth. Enzymol.,* 1987, vol. 155, 335-350
- **Altschul et al.** Gapped BLAST and PSI-BLAST: a new generation of protein database search programs. *Nucleic Acids Research,* 1997, vol. 25, 2289-3402

- **Esposti et al.** Critical evaluation of the hydropathy of membrane proteins. *Eur J Biochem,* 1990, vol. 190, 207-219